# EUROPEAN PATENT SPECIFICATION

(11) **EP 0 958 267 B1**
(45) Date of publication and mention of the grant of the patent: **31.03.2004**
(21) Application number: 97952830.4
(22) Date of filing: 25.11.1997
(51) Int. Cl.: C07C 31/125, C07C 43/13, C07C 305/06, C07C 53/126, C11D 1/72, C11D 1/14, C11D 1/29

(54) **HIGHLY BRANCHED PRIMARY ALCOHOL COMPOSITIONS, AND BIODEGRADABLE DETERGENTS MADE THEREFROM**
ZUSAMMENSETZUNG AUF BASIS VON HOCHVERZWEIGTEN PRIMÄREN ALKOHOLEN SOWIE DARAUS HERGESTELLTE BIOLOGISCH ABBAUBARE TENSIDE
COMPOSITIONS D'ALCOOL PRIMAIRE FORTEMENT RAMIFIE ET DETERGENTS BIODEGRADABLES FABRIQUES A PARTIR DE TELLES COMPOSITIONS

(30) Priority: 26.11.1996 US 755843; 26.11.1996 US 755827
(43) Date of publication of application: 24.11.1999
(73) Proprietor: SHELL INTERNATIONALE RESEARCH MAATSCHAPPIJ B.V., 2596 HR Den Haag (NL)
(72) Inventor: KRAVETZ, Louis, Houston, TX 77084 (US); MURRAY, Brendan, Dermot, Houston, TX 77077 (US); SINGLETON, David, Michael, Houston, TX 77024 (US)
(86) International application number: PCT/EP1997/006694
(87) International publication number: WO 1998/023566

(56) References cited:
- WO-A-85/02175
- FR-A- 1 151 630

## Description

### 1. Field of the Invention

The invention pertains to a new primary alcohol composition and the alkoxylates, sulfates and alkoxy sulfates thereof and to their production and uses.

### 2. Background of the Invention

The alcohols of long chain olefins, especially those having about 10 to 28 carbon atoms, have considerable commercial importance in a variety of applications including detergents, soaps, surfactants, and freeze point depressants in lubricating oils. These alcohols are produced by any one of commercial processes, such as the oxo or hydroformylation of long chain olefins. Typical long chain alcohols are the commercially available NEODOL alcohols made by Shell Chemical Company, the EXXAL alcohols available from Exxon Chemical, and the LIAL alcohols available from Enichem (NEODOL, EXXAL and LIAL are trade marks).

In the manufacture of the NEODOL alcohols, a predominantly linear olefin feed is subjected to hydroformylation by reacting carbon monoxide and hydrogen onto the olefin in the presence of an Oxo catalyst to form an alcohol. In excess of 80% of the number of alcohol molecules in the resultant alcohol composition are linear primary alcohols. Of the branched primary alcohols in the composition, substantially all, if not all, of the branching is on the C₂ carbon atom relative to the hydroxyl bearing carbon atom. These alcohols can subsequently be converted to anionic or nonionic detergents or general surfactants by sulfonation or ethoxylation, respectively, of the alcohol. Also known as anionic surfactants for detergents are the alcohol-ethoxysulfates.

The NEODOL line of alcohols has met with considerable commercial success with detergents because the NEODOL _alcohol compositions can be economically produced with high yields of linear alcohols. The desire to use linear - alcohols as intermediates for detergent grade surfactants exists because it is generally recognized that linear alcohols biodegrade, while the branched long chain alcohol sulfonates exhibit poor biodegradability. Since detergents and soaps used by consumers for washing are ultimately released into the environment, the need to provide a surfactant or detergent which biodegrades is well recognized.

For example, US 5,112,519 describes the manufacture of a surfactant by oligomerizing C₃ and C₄ olefins through a surface deactivated ZSM-23 catalyst to form oligomers, hydroformylating the oligomer, and recovering a semi-linear alcohol composition having less than 1.4 methyl branches, and whose branching is limited to methyl branches. The alcohol can be ethoxylated and/or sulfated and is reported to be biodegradable, and further have improved low temperature properties compared to isotridecyl alcohol. Retaining the linearity of the alcohol composition to less than 1.4, along with obtaining methyl branching were important considerations to achieving a biodegradable surfactant. It would be desirable, however, to obtain a biodegradable surfactant without limiting the branching to methyl branches, without limiting the branching to under 1.4, and without limiting oneself to a ZSM 23 surface deactivated catalyst. It would also be desirable to make a biodegradable surfactant without conducting oligomerization reactions through zeolite catalysts, which are expensive and may coke up or be used up quickly if one needs to build chain length through the catalyst.

Another product, EXXAL 13, is derived from propylene oligomerization through acid catalysis to a wide range of mono-olefins, the range having an average of 13 carbon atoms being distilled out, but containing some olefins in the C₁₀₋₁₅ range. The olefin is then subjected to hydroformylation using an oxo process. EXXAL 13 is reported to be a 3-4 methyl branched tridecyl-alcohol known for its use in lubricants and in those detergent formulations which do not require rapid biodegradation. This is because EXXAL 13 only slowly biodegrades. While such a high amount of branching is not necessary, it would be desirable to make a surfactant having a higher amount of branching for detergency which is nevertheless readily biodegradable.

US Patent 5,196,625 describes a dimerization process for producing linear and/or mono -branched C₁₀ to C₂₈ olefins using dimerization catalysts, for the production of biodegradable alkylbenzene sulfonates detergents by alkylating the olefins onto benzene. No mention is made of using the dimerized olefins to make alcohols.
Further, the patentee reported that it is generally recognized that "linear and mono-branched alkyl aromatic sulfonates are generally much more readily biodegraded than multibranched alkyl aromatic sulfonates and, hence, much more desirable as detergents." For this reason, the patentee sought to ensure that the olefins made were substantially linear and monobranched. Again, it would be desirable to make highly branched products that have good detergency and biodegradability from alcohols, and also without regard to limitations on the amount of branching being low.

The patentee of US 4,670,606 likewise recommended using "linear detergent oxo-alcohols or those in which the linear fraction is as,high as possible" for biodegradability reasons in the detergent field, while oxo-alcohols that are highly branched are desirable as lubricating oil additives because the branching depresses the freezing point of the lubrication oil. Thus, the invention was directed towards methods to separate the two from a mixture.

The desire to make highly linear high olefin alcohols was also expressed in US 5,488,174. In discussing the problems encountered by cobalt carbonyl catalyzed hydroformylation of olefins, the patentee noted that this process produced a composition which contained branched compounds when starting with internal olefins, which was particularly undesirable because of its poor biodegradability. Thus, the patentee recommended using catalytic processes which would produce mixtures exhibiting high linear/branching ratios.

As previously noted, the highly linear NEODOL alcohol line of intermediates for the production of detergent surfactants are commercially successful, in part because of their high linearity rendering them readily biodegradable. However, the high degree of linearity also increased the hydrophobicity of the hydrophobe part of the chain, thereby decreasing its cold water solubility/detergency. In general, the highly linear alcohol sulfates suffer from poor cold water solubility/detergency. Along with the concern for using biodegradable compounds, government regulations are also calling for the lowering of wash temperatures.

Thus, there exists a growing need for alcohol intermediates which are both biodegradable and exhibit good detergency at cold wash temperatures. The solution to this problem was not merely as simple as increasing the branching of the higher olefin alcohol in order to decrease hydrophobicity and thereby hopefully increase cold water detergency, because as noted above it is well recognized that branched compounds exhibit poor biodegradability.

### 3. Summary of the invention

There have now been found new compositions of branched primary alcohols and their alkoxylate, sulfate and alkoxy sulfate derivatives, which satisfy require-ments for biodegradability and cold water detergency, and processes for the production of these compositions.

The present invention therefore firstly relates to a branched primary alcohol composition, having from 11 to 36 carbon atoms and an average number of branches per molecule of from 0.7 to 3.0, said branching comprising methyl and ethyl branches, said composition comprising less than 0.5 atom % of quaternary carbon atoms.

The invention relates secondly to a process for preparing said branched primary alcohol composition, which process comprises the steps of:
a) contacting an olefin feed comprising a linear olefins having at least 10 carbon atoms with a catalyst effective for skeletally isomerising said linear olefin to yield a branched olefin of the same carbon number; and
b) converting said branched olefin to said primary alcohol composition.

The term "skeletal isomerisation", as used herein, refers to hydrocarbon isomerisation wherein straight chains are converted, at least in part, to branched chains of the same number of carbon atoms. The catalyst of step a) is preferably a zeolite having at least one channel with a crystallographic free diameter ranging from 0.42 to 0.70 nanometer. The conversion to alcohol in step b) is preferably by hydroformylation.

The invention relates thirdly to a different process for preparing said branched primary alcohol composition, having from 13 to 21 carbon atoms, which process comprises the steps of:
a) dimerising, in the presence of a homogeneous dimerisation catalyst, an olefin feed comprising a C₆-C₁₀ olefin to yield a C₁₂-C₂₀ branched olefin; and
b) Converting said C₁₂-C₂₀ branched olefin to said branched primary alcohol composition.

The olefin feed of step a) is preferably linear olefin and preferably comprises at least 85 wt% of C₆-C₁₀ olefins. Step a) is preferably a one-step dimerisation process. The homogenous catalyst preferably comprises a mixture of a nickel carboxylate or a nickel chelate, with an alkyl aluminium halide or an alkyl aluminium alkoxide. Optionally the branched olefin produced in step a) is subjected to a double-bond isomerisation step before embarking on step b). The conversion to alcohol in step b) is preferably by hydroformylation.

The invention relates in the fourth place to a branched primary alcohol alkoxylate composition, preparable by reacting said branched primary alcohol composition with an oxirane compound.

The invention relates in the fifth place to a branched primary alkyl sulphate, preparable by sulfating said branched a primary alcohol composition.
And in the sixth place the invention relates to a detergent composition comprising:
a) one or more surfactant(s) selected from the group of said branched primary alcohol alkoxylates, branched primary alkyl sulphates, and branched alkoxylated primary alkyl sulphates;
b) a builder; and
c) optionally one or more additives selected from the group of foam controlling agents, enzymes, bleaching agents, bleach activators, optical brighteners, co-builders, hydrotropes and stabilisers.

### 4. Detailed Description of the Invention.

As used herein, the phrase average number of branches per molecule chain refers to the average number of branches per alcohol molecule, as measured by ¹³C Nuclear Magnetic Resonance (¹³C NMR) as discussed below. The average number of carbon atoms in the chain are determined by gas chromatography.

Various references will be made throughout this specification and the claims to the percentage of branching at a given carbon position, the percentage of branching-based on types of branches, average number of branches, and percentage of quaternary atoms. These amounts are to be measured and determined by using a combination of the following three ¹³C-NMR techniques. (1) The first is the standard inverse gated technique using a 45-degree tip ¹³C pulse and 10 s recycle delay (an organic free radical relaxation agent is added to the solution of the branched alcohol in deuterated chloroform to ensure quantitative results). (2) The second is a J-Modulated Spin Echo NMR technique (JMSE) using a 1/J delay of 8 ms (J is the 125 Hz coupling constant between carbon and proton for these aliphatic alcohols). This sequence distinguishes carbons with an odd number of protons from those bearing an even number of protons, i.e. CH₃/CH vs CH₂/C_{q} (C_{q} refers to a quaternary carbon). (3) The third is the JMSE NMR "quat-only" technique using a 1/2J delay of 4 ms which yields a spectrum that contains signals from quaternary carbons only. The JSME NMR quat only technique for detecting quaternary carbon atoms is sensitive enough to detect the presence of as little at 0.3 atom% of quaternary carbon atoms. As an optional futher step, if one desires to confirm a conclusion reached from the results of a quat only JSME NMR spectrum, one may also runa DEPT-135 NMR sequence. We have found that the DEPT-135 NMR sequence is very helpful in differentiating true quaternary carbons from breakthrough protonated carbons. This is due to the fact that the DEPT-135 sequence produces the "opposite" spectrum to that of the JMSE "quat-only" experiment. Whereas the latter nulls all signals except for quaternary carbons, the DEPT-135 nulls exclusively quaternary carbons. The combination of the two spectra is therefore very useful in spotting non quaternary carbons in the JMSE "quat-only" spectrum. When referring to the presence or absence of quaternary carbon atoms throughout this specification, however, we mean that the given amount or absence of the quaternary carbon is as measured by the quat only JSME NMR method. If one optionally desires to confirm the results, then also using the DEPT-135 technique to confirm the presence and amount of a quaternary carbon.

The detergency evaluations conducted and as used throughout were based from a standard high density laundry powder (HDLP) Detergency/Soil Redeposition Performance test. The evaluations in the working examples were conducted using Shell Chemical Company's radiotracer techniques at the designated temperatures in Table III at a water hardness of 150 ppm as CaCO₃ (CaCl₂/MgCl₂ = 3/2 on a molar basis). The primary alcohol sulfated compositions of the invention were tested, on a 1/4 cup basis, against multisebum, cetanesqualane and clay soiled permanent press 65/35 polyester/cotton (PPPE/C) fabric. The HDLP's were tested at 0.74 g/l concentration, containing 27 wt% of the primary alcohol sulfate composition, 46 wt% of builder (zeolite-4A), and 27 wt% of sodium carbonate.

The composition of the radiolabeled Multisebum Soil was as follows:

| Component | Label | %wt. |
|---|---|---|
| Cetane | 3H | 12.5 |
| Squalane | 3H | 12.5 |
| Trisearin | 3H | 10 |
| Arachis (Peanut) | | |
| Oil | 3H | 20 |
| Cholesterol | 14C | 7 |
| Octadecanol | 14C | 8.0 |
| Oleic Acid | 14C | 15.0 |
| Stearic Acid | 14C | 15.0 |

A Terg-O-Tometer was used to wash the swatches at 15 minute intervals. The wash conditions were set to measure both cold water detergency at 10 °C and warm water detergency at 36 °C. The agitation speed was 100 rpm. Once the 4" x 4" radiotracer soiled swatches were washed by the Terg-O-Tometer, they were hand rinsed. The wash and rinse waters were combined for counting to measure sebum soil removal. The swatches were counted to measure clay removal.

For details concerning the detergency methods and radiotracer techniques, reference may be had to B.E. Gordon, H. Roddewig and W.T Shebs, HAOCS, 44:289 (1967), W.T. Shebs and B.E. Gordon, JAOCS, 45:377 (1968), and W.T. Shebs, Radioisotope Techniques in Detergency, Chapter 3, Marcel Dekker, New York (1987.

The biodegradation testing methods for measuring the biodegradability of the working example sulfates were conducted in accordance with the test methods established in 40 CFR §796.3200, also known as the OECD 301D test method. By a biodegradable primary alcohol sulfate composition or surfactant is meant that that the compound or composition gives a measured biochemical oxygen demand (BOD) of 60% or more within 28 days, and this level must be reached within 10 days of biodegradation exceeding 10 percent.

The primary alcohol composition of the invention contains an average chain length per molecule ranging from 11-36 carbon atoms. For many surfactant applications, such as detergents, the alcohol composition contains an average carbon chain length of 11, 12, 13, 14, 15, 16, 17, 18, 19, 20 or 21 carbon atoms, or any decimal inbetween, expressed as an average within the range of 11 to 21 carbon atoms. The number of carbon atoms includes carbon atoms along the chain backbone as well as branching carbons.

Preferably, at least 75 wt%, more preferably, at least 90 wt.% of the molecules in the primary alcohol composition have chain lengths ranging from 11 to 21, yet more preferably from 14 to 18 carbon atoms. As one feature of the invention, the average number of branches is at least 0.7, as defined and determined above. The compositions having an average number of branches of at least 1.5, in particular ranging from 1.5 to 2.3, especially from 1.7 to 2.1, achieve a good balance of cold water detergency and biodegradability when sulfated. Conventional linear alcohol sulfates contain an average number of branches of only 0.05 to 0.4, and are quite biodegradable. Up to this point, however, the introduction of a higher degree of branching for the purpose of improving cold water detergency has lead to failures in biodegradability tests. The primary alcohol composition of the invention, when sulfated, has the advantage of introducing a large number of branches to improve its cold water properties without sacrificing biodegradability. The cold water properties are improved when the amount of branching is at least 1.5.

A feature of the invention lies in the provision of a primary alcohol composition as defined above, having less than 0.5 atom% of C_{q}'s as measured by a quat only JMSE modified ¹³C-NMR having a detection limit of 0.3 atom% or better, and preferably an primary alcohol composition which contains no C_{q}'s as measured by this NMR technique. For reasons not yet clearly understood, it is believed that the presence of C_{q}'s on an alcohol molecule prevents the biodegradation of that particular sulfated molecule by biological organisms. Alcohols containing as little as 1 atom% of C_{q}'s have been been found to biodegrade at failure rates. It is also believed that previous attempts at the introduction of a high degree of branching has led to the formation of a sufficient number of C_{q}'s to account for biodegradation failure.

In a preferred embodiment of the invention, less than 5%, or more preferably less than 3%, of the alcohol molecules in the primary alcohol composition are linear alcohols. The efficient reduction in the number of linear alcohols to such a small amount in the composition results from introducing branching on an olefin feedstock by a skeletal isomerization or dimerisation technique using efficient catalysts as described further below, rather than introducing branching by methods such as acid catalyzed oligomerization of propylene molecules, or zeolite catalyzed oligomerization techniques. In a more preferable embodiment, the primary alcohol composition contains less than 3% of linear alcohols. The percentage of molecules which are linear may be determined by gas chromatography.

When the branching has been achieved by skeletal isomerization, the primary alcohol composition of the invention may be characterized by the NMR technique as having from 5 to 25% branching on the C₂ carbon position, relative to the hydroxyl carbon atom. In a more preferred embodiment, from 10 to 20% of the number of branches are at the C₂ position, as determined by the NMR technique. The primary alcohol composition also generally has from 10% to 50% of the number of branches on the C₃ position, more typically from 15% to 30% on the C₃ position, also as determined by the NMR technique. When coupled with the number of branches seen at the C₂ position, the primary alcohol composition in this case contain significant amount of branching at the C₂ and C₃ carbon positions.

Not only do the primary alcohol composition of the invention have a significant number of branches at the C₂ and C₃ positions, but we have also seen by the NMR technique that many of the primary alcohol compositions have at least 5% of isopropyl terminal type of branching, meaning methyl branches at the second to last carbon position in the backbone relative to the hydroxyl carbon. We have even seen at least 10% of terminal isopropyl types of branches in the primary alcohol composition, typically in the range of 10% to 20%. In typical hydroformylated olefins of the NEODOL series, less than 1%, and usually 0.0%, of the branches are terminal isopropyl branches. By skeletally isomerizing the olefin according to the invention, however, the primary alcohol composition contains a high percentage of terminal isopropyl branches relative to the total number of branches.

Considering the combined number of branches occurring at the C₂, C₃, and isopropyl positions, there are embodiments of the invention where at least 20%, more preferably at least 30%, of the branches are concentrated at these positions. The scope of the invention, however, includes branching occurring across the length of the carbon backbone. In another preferred embodiment of the invention, the total number of methyl branches number at least 40%, even at least 50%, of the total number of branches, as measured by the NMR technique described above. This percentage includes the overall number of methyl branches seen by the NMR technique described above within the C₁ to the C₃ carbon positions relative to the hydroxyl group, and the terminal isopropyl type of methyl branches.

Significantly, we have consistently observed a significant increase in the number of ethyl branches over those seen on NEODOL alcohols. The number of ethyl branches can range from 5% to 30%, most typically from 10% to 20%, based on the overall types of branching that the NMR method detects. Thus, the skeletal isomerization of the olefins produced both methyl and ethyl branches, and these alcohols, when sulfated, alkoxylated, or both, worked exceedingly well in biodegradability and detergency tests. Thus, the types of catalysts one may use to perform skeletal isomerization are not restricted to those which will produce only methyl branches. The presence of a variety of branching types is believed to enhance a good overall balance of properties.

The olefins used in the olefin feed for skeletal isomerization are at least C₁₀ mono-olefins. In a preferred range, the olefin feed comprises C₁₀ to C₃₅ mono-olefins. Olefins in the C₁₁ to C₁₉ range are considered most preferred for use in the instant invention, to produce primary alcohol compositions in the C₁₂ to C₂₀ range, which are the most common ranges for detergent applications. As a general rule, the higher the carbon number of the surfactant derivative, the more noticeable are the improvements in physical properties and formulateability.

In general, the olefins in the olefin feed composition are predominately linear. Attempting to process a predominately branched olefin feed, containing quaternary carbon atoms or extremely high branch lengths, would require separation methods after passing the olefin stream across the catalyst bed to separate these species from the desired branched olefins. While the olefin feed can contain some branched olefins, the olefin feed processed for skeletal isomerization preferably contains greater than about 50 percent, more preferably greater than about 70 percent, and most preferably greater than about 80 mole percent or more of linear olefin molecules.

The olefin feed generally feed does not consist of 100% olefins within the specified carbon number range, as such purity is not commercially available. The olefin feed is usually a distribution of mono-olefins having different carbon lengths, with at least 50 wt.% of the olefins being within the stated carbon chain range or digit, however specified. Preferably, the olefin feed will contain greater than 70 wt.%, more preferably about 80 wt.% or more of mono-olefins in a specified carbon number range (e.g., C₇ to C₉, C₁₀ to C₁₂, C₁₁ to C₁₅, C₁₂ to C₁₃, C₁₅ to C₁₈, etc.), the remainder of the product being olefin of other carbon number or carbon structure, diolefins, paraffins, aromatics, and other impurities resulting from the synthesis process.The location of the double bond is not limited. The olefin feed composition may comprise α-olefins, internal olefins, or a mixture thereof.

Chevron Alpha Olefin product series (trademark of and sold by Chevron Chemical Co.), manufactures predominantly linear olefins by the cracking of paraffin wax. Commercial olefin products manufactured by ethylene oligomerization are marketed in the United States by Shell Chemical Company under the trademark NEODENE and by Ethyl Corporation as Ethyl Alpha-Olefins. Specific procedures for preparing suitable linear olefins from ethylene are described in U.S. Patent Nos. 3,676,523, 3,686,351, 3,737,475, 3,825,615 and 4,020,121. While most of such olefin products are comprised largely of alpha-olefins, higher linear internal olefins are also commercially produced, for example, by the chlorination-dehydrochlorination of paraffins, by paraffin dehydrogenation, and by isomerization of alpha-olefins. Linear internal olefin products in the C₈ to C₂₂ range are marketed by Shell Chemical Company and by Liquichemica Company.

The catalyst used for skeletal isomerization preferably contains a zeolite having at least one channel with a crystallographic free channel diameter ranging from 0.42 to 0.70 nanometer, measured at room temperature, with essentially no channel present which has a free channel diameter which is greater than 0.70 nanometer.

The skeletal isomerization catalyst should contain at least one channel having a crystallographic free diameter at the entrance of the channel within the stated range. The catalyst should not have a diameter at the entrance of a channel which exceeds the 0.70 nanometer upper limit to the range. Zeolites possessing channel diamters greater than 0.7 nm are susceptible to unwanted aromatization, oligomerization, alkylation, coking and by-product formation. On the other hand, if the zeolite does not contain a channel having a free diameter along either of the x or y planes of at least 0.42 nm, the channel size becomes too small to allow diffusion of the olefin into and out of the channel pore once the olefin becomes branched. Thus, the zeolite must have at least one channel with free diameters of that channel within the range of 0.42 to 70 nm. All other specifications are preferences.

Without being bound to a theory, it is believed that the olefin molecule, due to its high carbon chain length, does not have to enter into the zeolite channel, diffuse through, and exit the other end of the channel. The rate of branching seen when passing the olefin feed across the zeolite does not correspond to the theoretical rate of branching if each olefin molecule were to pass through the channels. Rather, it is believed that most of the olefins partially penetrate the channel for a distance effective to branch the portion of the chain within the channel, and subsequently withdraw from the channel once isomerized. In this case, the olefin molecules in the composition would predominately have a structure which is branched at the ends of the olefin carbon backbone, and substantially linear towards the center of the molecule, i.e., at least 25% of the carbons at the center are unbranched. The scope of the invention, however, includes branching anywhere along the carbon backbone within the parameters described above with respect to the molecular structure.

Preferred embodiments of the zeolite structure are described in U.S. Patent No. 5,510,306. Zeolite structures are also described in the Atlas of Zeolite Structure Types, by W.M. Meier and D.H. Olson. With respect to structure, in a preferred embodiment, the catalyst contains a channel having free diameters within the range of greater than 0.42 nm to less than 0.70 nm long both the x and y planes in the [001] view. Zeolites with this specified channel size are typically referred to as medium or intermediate channel zeolites and typically have a 10-T member (or puckered 12-T member) ring channel structure in one view and a 9-T member or less (small pore) in another view, if any. There is no limit to the number of channels or their orientation (parallel, non-interconnecting intersections, or interconnecting at any angle) in the zeolite. There is also no limit to the size of the channels which are outside of the stated range in both the x and y planes, so long as these other channels do not have free diameter in either of the x or y planes which is greater than 0.70 nm. For example, other channels having a free diameter of 0.42 nm or less in one or both of the x or y are within the scope of the invention.

There is also no limit on the number of dimensions, one, two, or three, that the channel system may have. While the scope of the invention includes two or three dimensional zeolites with interconnecting channels having any size less than 0.70 nm, and including at least one channel within the stated range, there may exist limited circumstances where, for example, α-olefins may meet at the intersection of the interconnecting channels and dimerize or oligomerize, depending on the size of the olefin, the proximity of the interconnecting intersection to the channel entrances, the size of the interconnecting intersection, temperature, flow rates, among other factors. While it is unlikely that such dimer could diffuse back out of the zeolite, the dimer may coke the catalyst or crack within the channel structure, forming by-product olefins having quaternary carbon branching. Thus, the interconnecting channel system in a two or three dimensional zeolite should have free diameters effective to prevent the formation of dimers, trimers, or oligomers under the given processing conditions, which when cracked, can produce quaternary branched byproducts. In a preferred embodiment, all channels interconnecting to the channel within the stated range have free diameters in both of the x and y planes of 0.42 nm or less in order to eliminate the possibility that two olefin molecules would contact each other within the zeolite and dimerize or trimerize. This preference, however, applies only to interconnecting channels. A zeolite containing more than one channel, whether one, two, or three dimensional or even intersecting on different planes, but none of which interconnect, does not raise the possibility of dimerization or trimerization because the channels do not connect. Thus, there is no preference for these types of structures, so long as the basic requirements noted above are observed.

Examples of zeolites, including molecular sieves, that can be used in the processes of this invention with a channel size between 0.42 and 0.70 nm, include ferrierite, AlPO-31, SAPO-11, SAPO-31, SAPO-41, FU-9, NU-10, NU-23, ZSM-12, ZSM-22, ZSM-23, ZSM-35, ZSM-48, ZSM-50, ZSM-57, SUZ-4A, MeAPO-11, MeAPO-31, MeAPO-41, MeAPSO-11, MeAPSO-31, and MeAPSO-41, MeAPSO-46, ELAPO-11, ELAPO-31, ELAPO-41, ELAPSO-11, ELAPSO-31, and ELAPSO-41, laumontite, cancrinite, offretite, hydrogen form of stilbite, the magnesium or calcium form of mordenite and partheite. The isotypic structures of these frameworks, known under other names, are considered to be equivalent. An overview describing the framework compositions of many of these zeolites is provided in New Developments in Zeolite Science Technology, "Aluminophosphate Molecular Sieves and the Periodic Table," Flanigen et al. (Kodansha Ltd., Tokyo, Japan 1986).

Many natural zeolites such as ferrierite, heulandite and stilbite feature a one-dimensional pore structure with a pore size at or slightly smaller than about 0.42 nm diameter. These same zeolites can be converted to zeolites with the desired larger channel sizes by removing the associated alkali metal or alkaline earth metal by methods known in the art, such as ammonium ion exchange, optionally followed by calcination, to yield the zeolite in substantially its hydrogen form. See e.g., U.S. Pat. Nos. 4,795,623 and 4,942,027. Replacing the associated alkali or alkaline earth metal with the hydrogen form correspondingly enlarges the channel diameter. It is understood that the channel diameter or "size" shall mean the effective channel diameter or size for diffusion. Alternatively, natural zeolites with too large a channel size, such as some forms of mordenite, can be altered by substituting the alkali metal with larger ions, such as larger alkaline earth metals to reduce the channel size and thus become useful for the processes of this invention.

Particularly preferred zeolites are those having the ferrierite isotypic framework structure (or homeotypic). See the Atlas of Zeolite Structure Types, by W. M. Meier and D. H. Olson, published by Butterworth-Heinemann, third revised edition, 1992, page 98. The prominent structural features of ferrierite found by x-ray crystallography are parallel channels in the alumino-silicate framework which are roughly elliptical in cross-section. Examples of such zeolites having the ferrierite isotypic framework structure include natural and synthetic ferrierite (can be orthorhombic or monoclinic), Sr-D, FU-9 (EP B-55,529), ISI-6 (U.S. Pat. No. 4,578,259), NU-23 (E.P. A-103,981), ZSM-35 (U.S. Pat. No. 4,016,245) and ZSM-38 (U.S. Pat. No. 4,375,573). The hydrogen form of ferrierite (H-ferrierite) is the most preferred zeolite and considered to be substantially one-dimensional, having parallel running channels, with elliptical channels having free diameters of 0.42 x 0.54 nm along the x and y planes in the [001] view, which is large enough to permit entry of a linear olefin and diffusion out of or through the channel of the methyl branched isoolefin and small enough to retard coke formation. Methods for preparing various H-ferrierite are described in U.S. Pat. Nos. 4,251,499, 4,795,623 and 4,942,027.

The skeletal isomerization catalyst used in the isomerization processes of this invention may be combined with a refractory oxide that serves as a binder material. Suitable refractory oxides include natural clays, such as bentonite, montmorillonite, attapulgite, and kaolin; alumina; silica; silica-alumina; hydrated alumina; titania; zirconia and mixtures thereof. The weight ratio of zeolite to binder material suitably ranges from 10:90 to 99.5:0.5, preferably from 75:25 to 99:1, more preferably from 80:20 to 98:2 and most preferably from 85:15 to 95:5 (anhydrous basis).

Preferably the binder is, for example, selected from the silicas, the aluminas, the silica-aluminas and the clays. More preferred binders are aluminas; such as pseudoboehmite, gamma and bayerite aluminas. These binders are readily available commercially and are used to manufacture alumina-based catalysts. LaRoche Chemicals, through its VERSAL family of aluminas and Vista Chemical Company, through its CATAPAL aluminas, provide suitable alumina powders which can be used as binders in preparing the instant catalysts (VERSAL and CATAPAL are trade marks). Preferred alumina binders to be used in the preparation of the catalyst, particularly when extrusion is utilized, are the high-dispersity alumina powders. Such high-dispersity aluminas have a dispersity of greater than 50% in a aqueous acid dispersion having an acid content of 0.4 milligram equivalents of acid (acetic) per gram of Al₂O₃. Such high-dispersity aluminas are exemplified by Vista's CATAPAL D alumina.

Preferably, the skeletal isomerization catalyst is also prepared with at least one acid selected from monocarboxylic acids and inorganic acids and at least one organic acid with at least two carboxylic acid groups ("polycarboxylic acid"). Preferred monocarboxylic acid includes monocarboxylic acid having substituted or unsubstituted hydrocarbyl group having 1 to 20 carbon atoms which can be aliphatic, cyclic or aromatic. Examples include acetic acid, formic acid, propionic acid, butyric acid, caproic acid, glycolic acid, lactic acid, hydroxylbutyric acid, hydroxycyclopentanoic acid, salicylic acid, mandelic acid, benzoic acid, and fatty acids. Preferred inorganic acid includes mineral'acids such as nitric acid, phosphoric acid, sulfuric acid and hydrochloric acid.

The preferred polycarboxylic acid is an organic acid with two or more carboxylic acid groups attached through a carbon-carbon bond linkage to an hydrocarbyl segment. The linkage can be at any portion of the hydrocarbyl segment. The polycarboxylic acid preferably has an hydrocarbyl segment from 0 to 10 carbon atoms which can be aliphatic, cyclic or aromatic. The hydrocarbyl segment has 0 carbon atoms for oxalic acid with two carboxylic acid groups attached through the carbon-carbon bond. Examples of the polycarboxylic acids includes, for example, tartaric acid, citric acid, malic acid, oxalic acid, adipic acid, malonic acid, galactaric acid, 1,2-cyclopentane dicarboxylic acid, maleic acid, fumaric acid, itaconic acid, phthalic acid, terephthalic acid, phenylmalonic acid, hydroxyphtalic acid, dihydroxyfumaric acid, tricarballylic acid, benzene-1,3,5-tricarboxylic acid, isocitric acid, mucic acid and glucaric acid. The polycarboxylic acids can be any isomers of the above acids or any stereoisomers of the above acids. Polycarboxylic acids with at least two carboxylic acid groups and at least one hydroxyl group is more preferred. The most preferred second acids (i.e., polycarboxylic acids) are citric acid, tartaric acid and malic acid.

Optionally, coke oxidation promoting metals can be incorporated into the instant catalysts to promote the oxidation of coke in the presence of oxygen at a temperature greater than about 250 °C. While the term "metal(s)" is used herein in reference to the oxidation catalysts, these metals will not necessarily be in the zero-valent oxidation state,and in many cases will be in the higher oxidation states. Thus, "metal(s)" can encompass the oxides as well as the metals. Preferably the coke oxidation-promoting metal(s) used are transition and rare earth metals. More preferably the coke oxidation-promoting metals are selected from Groups IB, VB, VIB, VIIB and VIII of the transition metal series of the Periodic Table. Specifically preferred are Pd, Pt, Ni, Co, Mn, Ag and Cr. Most preferred are the Group VIII metals palladium and/or platinum. The amount of metal introduced can be up to about 2% by weight, measured as the metal per total weight of the catalyst. When using platinum and/or palladium, smaller rather than larger amounts of metals incorporated into the zeolite/binder are preferred. Preferably platinum and/or palladium will range from 5 to 3000 ppm by weight, basis metal, of the final catalyst.

In a preferred method, the instant catalysts can be prepared by mixing a mixture of at least one zeolite as herein defined, alumina-containing binder, water, at least one monocarboxylic acid or inorganic acid and at least one polycarboxylic acid in a vessel or a container, forming a pellet of the mixed mixture and calcining the pellets at elevated temperatures. In one preferred embodiment zeolite powder and alumina-containing powder is mixed with water and one or more of monocarboxylic acid or inorganic acid (first acid) and one or more of polycarboxylic acid (second acid) and optionally one or more compounds of the coke-oxidation promoting metal and the resulting mixture (paste) is formed into a pellet. The coke-oxidation promoting metal may alternatively be impregnated.

Preferably the pellet is formed by extrusion but can also be formed into catalytically useful shape by pressing hydrostatically or mechanically by pressing into die or mold. When extrusion is used optional extrusion aids such as cellulose derivatives, e.g., METHOCEL F4M hydroxypropyl methylcellulose (METHOCEL is a trade mark), can be utilized (manufactured by The Dow Chemical Company). The term "pellets" as used herein can be in any shape or form as long as the materials are consolidated. The formed pellets are calcined at a temperature ranging from a lower range of from 200 °C., preferably from 300 °C., more preferably from 450 °C, to an upper range of up to 700 °C., preferably up to 600 °C., more preferably up to 525 °C.

The ratio of the first acids to second acids is preferably within the range of 1:60 to 60:1, more preferably 1:10 to 10:1. The amount of the first acid used is in an amount effective to peptize the mixture. Preferably the amount of the first acid used is from 0.1 to 6 weight percent, more preferably from 0.5 to 4 weight percent based on the combined weight of zeolite and alumina-containing binder (anhydrous solids basis). Aluminas with lower dispersibilities than Vista CATAPAL D may require greater amounts of acid to peptize them. The amount of the second acid used is in an amount effective to promote the catalytic activity of the catalyst which is from 0.1 to 6, preferably from 0.2 to 4 weight percent based on the combined weight of zeolite and alumina-containing binder (anhydrous solids basis).

The mixture is mixed thoroughly or vigorously until the mixture appears uniform. The mixing can be performed by combining all of the components of the mixture at once or by adding the components of the mixture at different stages of mixing. The mixing can be accomplished by mulling. The term "mulling" is used herein to mean mixing of powders to which sufficient water has been added to form a thick paste and wherein the mixing is accompanied by shearing of the paste. Commercially available mullers such as the Lancaster Mix Muller and the Simpson Mix Muller can be used to carry out the mixing. A commercial blender such as a ribbon blender and/or a powder mill can also be used to carry out the mixing.

Optionally the coke-oxidation promoting metal can be impregnated to the formed pellet with a metals-containing solution instead of mixing in the paste mixture.

The temperature at which the skeletal isomerization may be conducted may range from 200 °C to 500 °C. Temperatures should not exceed the temparture at which the olefin will crack. Suitable pressures maintained during the isomerization reaction is at an olefin partial pressure ranging from 10 to 1000 kPa, more preferably from above 50 to 500 kPa, most preferably above 50 to 200 kPa.

High conversion, high selectivity, and high yields are attained by the process of the present invention. The olefin feed comprising linear olefins having an average of at least 7 carbon atoms are contacted with a catalyst effective for skeletally isomerizing said linear olefins at a conversion of at least 40% in a single pass. However, the process of the invention enables one to skeletally isomerize the linear olefins at far higher conversions. Conversions of at least 70%, preferably at least 80%, more preferably at least 90%, and most preferably at least 95% are attainable in a single pass in the process of the invention. Advantageously, the conversion percentages are obtainable at temperatures ranging from 200 °C to 500 °C, preferably at temperatures ranging from 200 °C to 350 °C.

The catalyst employed in the skeletal isomerization process is also highly selective towards the manufacture of skeletally isomerized branched olefins. Although high selectivities are attainable according to the skeletal isomerization process, the selectivity of the catalyst towards the manufacture of C7 or higher branched olefins from a linear C7 or higher linear olefin stream is at least 30% in one pass since processes using catalysts with lower selectivities are not regarded as sufficiently effective or economically justified. Higher selectivities of at least 70%, more preferably at least 80%, most preferably at least 90%, and even as high as at least 95%, are attainable in the process of the invention in one pass.

Another advantage observed by skeletally isomerizing the C7 or higher linear olefin streams according to the process of the invention is that high conversion of the linear olefin stream can be obtained in conjunction with high selectivity to the skeletally isomerized branched olefins. The olefin stream is preferably converted at percentages of at least 70%, with a selectivity to the skeletally isomerized branched olefins of at least 70%, more preferably at least 80%, most preferably at least 90%. In another embodiment, at least 80% of the olefin stream is converted, at a selectivity to the skeletally isomerized branched olefins of at least 80%, more preferably a selectivity of at least 90%, most preferably at least 95% selectivity. In a further embodiment, at least 90% of the olefins are converted at selectivities of 90% or more toward the skeletally isomerized branched olefins, more preferably at selectivities of 95% or more.

The skeletal isomerization process also produces high yields of skeletally isomerized branched olefins. The yield of the skeletally isomerized branched olefins should be at least about 10%, but yields of at least about 50%, more preferably at least about 65%, most preferably,at least about 80%, and even as high as at least about 90% are achievable in practice. The highest yield of skeletally isomerized branched olefin obtained is limited by the equilibrium concentration of the branched olefins at the skeletal isomerization temperature.

The present skeletal isomerization process can be operated at a wide range of conditions. Olefin weight hour space velocity of the olefin can range from 0.1 to 100 per hour. Preferably, the WHSV is between 0.5 to 50, more preferably between 1 and 40, most preferably between 2 and 30 per hour. At lower WHSV's, it is possible to operate at lower temperatures while achieving high yields of skeletally isomerized branched olefins. At higher WHSV's, the temperature is generally increased in order to maintain the desired conversion and selectivity to the skeletally isomerized branched olefins. Further, optimal selectivities are generally achieved at lower olefin partial pressures mentioned above. For this reason, it is often advantageous to dilute the feed stream with a diluent gas such as nitrogen or hydrogen. Although reducing the olefin partial pressure with a diluent may be beneficial to improve the selectivity of the process, it is not necessary to dilute the olefin stream with a diluent.

If a diluent is used, the molar ratio of olefin to diluent can range from 0.01:1 to 100:1, and is generally within the range of 0.1:1 to 5:1.

When the branching has been achieved by dimerisation, the primary alcohol composition of the invention have relatively few branch points at the C₁ through C₃ carbon positions relative to the hydroxyl carbon and little or no isopropyl terminations, that is, little or no branches at the second to last carbon atom along the backbone of the alcohol molecule relative to the hydroxyl carbon atom. In particular, the typical alcohol molecule in this case contains less than 25% branching at the C₂ and C₃ positions, and less than 5% isopropyl termination, more usually no isopropyl groups being detected.

From these carbon positions, the alcohol molecules made from the dimerised olefins look similar to the NEODOL alcohols. However, unlike the NEODOL alcohols which are predominately linear, the primary alcohol composition of the invention has a very high average number of branches per molecule. Due to large number of branches found in the primary alcohol composition of the invention and the relatively low percentage of branch points at the C₂, C₃, and isopropyl terminal carbon positions, the majority of the branches are toward the center of the molecule, with a significant number of the branches being located on one or both of the dimerized carbon atoms. The NMR spectral data is consistent with where the branches are thought to be located based on a chemical reaction equation.

The types of branching found in the primary alcohol composition of the invention varies from methyl, ethyl, propyl, and butyl or higher. A significant number of the branches detected by the NMR were ethyl groups, although this can vary depending upon the composition of the feed and reaction conditions. In one embodiment, however, the number of ethyl groups in the primary alcohol composition of the invention range, preferably, from 10 % to 30%, which is a significant jump from the amount of ethyl groups detected in NEODOL alcohols. The number of methyl groups detected by the NMR can also vary widely for the same reason. Typically, however, the number of methyl groups will range from 10% to 50%, as detected by the NMR.

Broadly speaking, a primary alcohol composition is obtained by dimerizing an olefin feed comprising C₆-C₁₀ linear olefins in the presence of a dimerization catalyst under dimerization conditions to obtain C₁₂-C₂₀ olefins.

In one embodiment, the olefin feed comprises at least 85 mol%, preferably at least 90 mol%, more preferably at least 95 mol% linear olefins. The remainder of the olefin feed comprises only a small number of branched olefins, preferably less than 3 mol%.

The olefin feed can contain shorter or longer olefins. In a preferred embodiment, however, the olefin stream also comprises at least 85 wt% of the C₆-C₁₀ olefins, more preferably 90 wt%, and most preferably 95 wt% C₆-C₁₀ olefins. Another advantage of the process of the invention is that one can make mixtures of both odd and even numbered dimerized olefins by employing mixtures of both odd and even numbered olefins in the feed, as distinguished from those processes which rely upon oligomerizing either C₃ or C₄ olefins to build higher olefins.

The olefin feed can be made up of internal or alpha olefins, or mixtures thereof. Preferably, the majority of the olefins present in the feed comprise internal olefins because the dimerization of these olefins tend to produce a variety of branch types, that is, methyl, ethyl, and propyl branches, even butyl branches. By a majority is meant that greater than 50 wt% of the olefin feed is comprised of internal olefins. More preferably, at least 75 wt% of the olefin feed is comprised of internal olefins.

In one embodiment of the invention, there is provided a one-step dimerization process.

By a one-step dimerization process is meant that an olefin feed, once dimerized, is not further subjected to dimerization. A one-step process does, however, include recycling unreacted olefin feed to the dimerization zone because this unreacted olefin was not dimerized. It also includes a continuous process or several batch reactors operating in parallel, so long as one dimerized stream of olefin is not fed to a subsequent dimerization reaction zone for a second or subsequent dimerization. This one step process provides the advantage that one may use conventional streams of olefins without the necessity for expensive and sophisticated extraction and separation processes for making an olefin stream of high purity linear olefins. The olefin feed may be obtained by the conventional oligomerization of ethylene, which may subsequently be disproportionated, or by the Fischer-Tropsch process, which uses a 1 carbon oligomerization by passing CO and H₂ over iron or cobalt catalyst; as distinguished from trimer or tetramer feeds of C₃ or C₄ olefins which are highly branched or require special extraction steps to obtain high linearity in the olefin feed.

Typically, the dimerization is conducted at temperatures in the range of from about -10 °C to 100 °C., preferably from 20° to 50 °C for the duration of 1/2 to 8 hours, preferably 1 to 5 hours, using an olefin to catalyst mole ratio of 200 to 20,000, preferably 1,000 to 10,000 moles of olefin per mole of catalyst. The dimerization is generally conducted as a liquid phase reaction using pressures in the range of 0 to 300 kPa, preferably 100 to 200 kPa. Where the dimerization is conducted as a batch process, the catalyst can be conveniently prepared in situ in the reactor. The dimerization can also be conducted as a continuous, semi-batch or multi-step process. It should be appreciated that where typical or preferred process conditions (e.g., temperatures, times, catalyst ratios, etc.) have been given, that other process conditions could also be used. Optimum reaction conditions (e.g., temperature, reaction time, reactant ratios, catalyst ratios, solvents, etc.) may vary with the particular reactants, catalysts, or solvents used, but can be determined by routine optimization procedures.

The dimerization catalysts of this invention can be prepared by contacting the appropriate components of the catalyst in the olefin to be dimerized. Preferably, the components of the catalyst are not mixed together prior to their addition to the olefin feed, as this may cause decomposition of the catalyst. Added solvents, such as chlorobenzene or cyclohexane may be used and do not detract from catalyst performance.

The selection of the catalyst for the dimerization is one which is selective towards the manufacture of high yields of dimerized olefins having and average of from 0.7 to 3.0 and preferably 0.9 to 2.0 branches per molecule. These catalysts are preferably soluble in hydrocarbon media, for example, the olefin feed stream. Examples of dimerization catalysts soluble in hydrocarbons are complexes wherein a metal, preferably nickel, is bound to at least one hydrocarbon group, for example a bis- nickel, a nickel halide or bis-cyclooctadiene nickel associated to a halogenated aluminium compound. Another type of catalyst consists of the complexes formed by admixing at least one nickel compound with at least one alkylaluminium compound and optionally a ligand, for example a phosphine. These catalysts are well-known in the art. Illustrations of the catalyst that can be used in this type of process are given in U.S. Patent Nos. 4,366,087; 4,326,650; and 4,398,049.

A preferred class of catalysts used in the process are homogenous catalysts comprising a combination of a nickel carboxylate or a nickel chelate, with an alkyl aluminium halide or an alkyl aluminium alkoxide, respectively. The Al/Ni mole ratio is suitably from 1.0 to 20.0.

The nickel compound comprises a nickel carboxylate or a nickel chelate. The carboxylate of the nickel carboxylate may be represented by the formula (RCOO)₂Ni, where R is a branched or unbranched, hydrocarbyl radical, for example an alkyl, cycloalkyl, alkenyl, aryl, aralkyl or alkaryl radical, containing at least 2 carbon atoms, preferably a sufficient number of carbon atoms to render it compatible with hydrocarbon media, such as a hydrocarbyl radical of 5-20 carbon atoms, which radical may be substituted with, for example-, hydroxy groups. One of the RC00- groups of the bivalent nickel carboxylate mentioned above may optionally be substituted with group represented by R₁COO-, where R₁ is a halogenoalkyl radical containing from 1 to -3 carbon atoms, as described in US Patent No. 4,366,087.

Examples of the nickel carboxylates include, but are not limited to, bis-(2-ethylhexanoate)nickel; 2-ethylhexanoate nickel trichloro(or trifluoro) acetate; 2-ethylhexanoate nickel o-chlorobenzoate; and 2-ethylhexanoate nickel acetylacetonate, nickel 2-ethylbutyrate trifluoroacetate, nickel 2-ethylbutyrate trichloroacetate, nickel 3,3-dimethylbutyrate trifluoroacetate, nickel 3,3-dimethylbutyrate trichloroacetate, nickel 4-methylvalerate trifluoroacetate, nickel heptanoate trifluoroacetate, nickel heptanoate trichloroacetate, nickel heptanoate tribromoacetate, nickel heptanoate triiodoacetate, nickel 2-ethylhexanoate monofluoroacetate, nickel 2-ethylhexanoate trichloroacetate, nickel 2-ethylhexanoate dichloroacetate, nickel 2-ethylhexanoate monochloroacetate, nickel 2-ethylhexanoate tribromoacetate, nickel 2-ethylhexanoate triiodoacetate, nickel octoate trifluoroacetate, nickel octoate trichloroacetate, nickel decanoate trifluoroacetate, nickel decanoate trichloroacetate, nickel myristate trifluoroacetate, nickel palmitate trifluoroacetate, nickel dodecylbenzoate trifluoroacetate, nickel diisopropylsalicylate trichloroacetate, nickel myristate pentafluoropropionate and nickel 2-ethylhexanoate heptafluorobutyrate.
The nickel chelate compounds, which react with the alkyl aluminium alkoxides, are described in US Patent Nos. 3,424,815 and 4,959,491. The nickel chelates include those having the formula 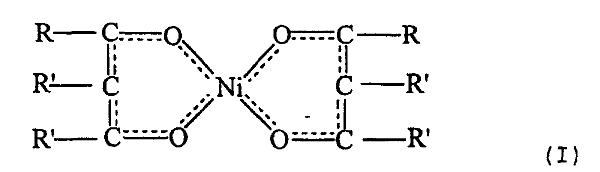 wherein R and R' independently are hydrogen, alkyl or aryl of up to 10 carbon atoms, or haloalkyl or haloaryl of up to 10 carbon atoms, with the proviso that the two R' groups of each chelating ligand together with the adjacent carbon atoms to which they are attached, can form a six-membered carbocyclic aromatic ring of up to 4 halogen substituents. The halogenated chelating ligand preferably has up to 15 carbon atoms and from 2 to 8 halogen substituents, but more preferably has up to 10 carbon atoms and from 3 to 6 halogen substituents. The halogen substituents of the chelating ligand are suitably fluorine, chlorine, bromine or iodine, wherein the R' groups together form a divalent radical in which the monoenol configuration is maintained as part of the aromatic ring;

The aluminium compound comprises an hydrocarbyl aluminium halide or a hydrocarbyl aluminium alkoxide. The hydrocarbyl group generally comprises 0, 1 or 2 hydrocarbyl groups each having from 1 to 20 carbon atoms, usually from 1 to 12 carbon atoms, the groups including alkyl, aryl, aralkyl, alkaryl, and cycloalkyl. The halide comprises 1 to 6 halides, such as fluoride, iodide, chloride, or bromide, preferably whichever is readily available, such as the chloride. Examples of the hydrocarbyl aluminium halides include AlCl₃, ethylaluminium dichloride, ethylaluminium sesquichloride, dichloroethylaluminium, dichloroisobutylaluminium, chlorodiethylaluminium or their mixtures.

Suitable alkoxides can be 1 or 2 alkoxide groups whose alkyl segments are as defined above with respect to the alkyl groups attached to the aluminium.

Optionally, the catalyst may also contain a small amount of water which has the effect of increasing the rate of the catalytic dimerization. Generally, the amount of water employed will be an amount sufficient to increase the rate of the catalytic dimerization.

At the outlet of the reactor, the catalyst may be deactivated in known manner, for example, with ammonia and/or an aqueous sodium hydroxide solution and/or an aqueous sulfuric acid solution, or an organic acid/bicarbonate solution. The unconverted olefins and the alkanes, if any, are separated thereafter from the oligomers by distillation, or any other suitable procedure, such as extraction, and the like. Unreacted feedstock can be recycled back to the initial feedstock.

The branched - skeletally isomerized or dimerized - olefins are subsequently converted to alcohols and to any of a broad range of surfactants, including nonionic, anionic, cationic, and amphoteric surfactants. The branched olefin serves as a surfactant intermediate. Specifically, the branched olefin serves as the hydrophobic moiety of the surfactant molecule, while the moiety added to the olefin during the conversion process serving as the hydrophile. Neither the particular surfactant nor the means used to convert the branched olefin to an alcohol or surfactant is considered critical to the present invention, provided that it does not rearrange the skeletal structure of the olefin to the extent that the byproduct is no longer biodegradable, or - reduces the degree of branching to less than 0.7.

Conversion of the branched olefins to a primary alcohol composition is conveniently accomplished, for example, by hydroformylation, by oxidation and hydrolysis, by sulfation and hydration, by epoxidations and hydration, or the like. In hydroformylation, the skeletally isomerized olefins are converted to alkanols by reaction with carbon monoxide and hydrogen according to the Oxo process. Most commonly used is the "modified Oxo process", using a phosphine, phosphite, arsine or pyridine ligand modified cobalt or rhodium catalyst, as described in U.S. Patent Nos. 3,231,621; 3,239, 566; 3,239,569; 3,239,570; 3,239,571; 3,420,898; 3,440,291; 3,448,158; 3,448,157; 3,496,203; and 3,496,204; 3,501,515; and 3,527,818. Methods of production are also described in Kirk Othmer, "Encyclopedia of Chemical Technology" 3^{rd} Ed. vol 16, pages 637-653; "Monohydric Alcohols: Manufacture, Applications and Chemistry", E. J. Wickson, Ed. Am. Chem. Soc. 1981.

Hydroformylation is a term used in the art to denote the reaction of an olefin with CO and H₂ to produce an aldehyde/alcohol which has one more carbon atom than the reactant olefin. Frequently, in the art; the term hydroformylation is utilized to cover the aldehyde and the reduction to the alcohol step in total, i.e., hydroformylation refers to the production of alcohols from olefins via carbonylation and an aldehyde reduction process. As used herein, hydroformylation refers to the ultimate production of alcohols.

Illustrative catalysts include cobalt hydrocarbonyl catalyst, cobalt-phosphine ligand catalyst, and rhodium-phosphine ligand catalyst. The choice of catalysts determines the various reaction conditions imposed. These conditions can vary widely, depending upon the particular catalysts. For example, temperatures can range from about room temperatures to 300 °C. When cobalt carbonyl catalysts are used, which are also the ones typically used, temperatures will range from 150° to 250 °C. One of ordinary skill in the art, by referring to the above-cited references, or any of the well-known literature on oxo alcohols can readily determine those conditions of temperature and pressure that will be needed to hydro-formylate the dimerized olefins.

Typical reaction conditions, however, are moderate. Temperatures in the range of 125 °C to 200 °C are recommended. Reaction pressures in the range of 2170 to 10440 kPa are typical, but lower.or higher pressures may be selected. Ratios of catalyst to olefin ranging from 1:1000 to 1:1 are suitable. The ratio of hydrogen to carbon monoxide can vary widely, but is usually in the range of 1 to 10, preferably about 2 moles of hydrogen to one mole of carbon monoxide to favor the alcohol product.

The hydroformylation process can be carried out in the presence of an inert solvent, although it is not necessary. A variety of solvents can be applied such as ketones, e.g. acetone, methyl ethyl ketone, methyl isobutyl ketone, acetophenone and cyclohexanone; aromatic compounds such-as benzene, toluene and the xylenes; halogenated aromatic compounds such as chlorobenzene and orthodichlorobenzene; halogenated paraffinic hydrocarbons such as methylene chloride and carbon tetrachloride; paraffins such as hexane, heptane, methylcyclohexane and isooctane and nitriles such as benzonitrile and acetonitrile.

With respect to the catalyst ligand, mention may be made of tertiary organo phosphines, such as trialkyl phosphines, triamyl phosphine, trihexyl phosphine, dimethyl ethyl phosphine, diamylethyl phosphine, tricyclopentyl(or hexyl) phosphine, diphenyl butyl phosphine, dipenyl benzyl phosphine, triethoxy phosphine, butyl diethyoxy phosphine, triphenyl phosphine, dimethyl phenyl phosphine, methyl diphenyl phosphine, dimethyl propyl phosphine, the tritolyl phosphines and the corresponding arsines and stibines. Included as bidentate-type ligands are tetramethyl diphosphinoethane, tetramethyl diphosphinopropane, tetraethyl diphosphinoethane, tetrabutyl diphosphinoethane, dimethyl diethyl diphosphinoethane, tetraphenyl diphosphinoethane, tetraperfluorophenyl diphosphinoethane, tetraphenyl diphosphinopropane, tetraphenyl diphosphinobutane, dimethyl diphenyl diphosphinoethane, diethyl diphenyl diphosphinopropane - and tetratrolyl diphosphinoethane.

Examples of other suitable ligands are the phosphabicyclohydrocarbons, such as 9-hydrocarbyl-9-phosphabicyclononane in which the smallest P-contianing ring contains at least 5 carbon atoms. Some examples include 9-aryl -9-phosphabicyclo[9.2.1]nonane, (di)alkyl-9-aryl - 9-phosphabicyclo[4.2.1]nonane, 9-alkyl -9-phosphabicyclo[4.2.1]nonane, 9-cycloalkyl -9-phosphabicyclo[4.2.1]nonane, 9-cycloalkenyl -9-phosphabicyclo[4.2.1]nonane, and their [3.3.1] and [3.2.1] counterparts, as well.as their triene counterparts.

The branched primary alcohol composition of the invention is suitable for the manufacture of anionic, nonionic, and cationic surfactants, preferably the former two, more preferably the anionic. Specifically, the branched primary alcohol composition of the invention can be used as the pecursor for the manufacture of anionic sulfates, including alcohol sulfates and oxylakylated alcohol sulfates, and nonionic oxyalkylated alcohols.

Any technique known for sulfating alcohols can be used herein. The primary alcohol composition may be directly sulfated, or first oxyalkylated followed by sulfatation. A preferred class of compositions comprises at least one anionic surfactant comprising the condensation product of the C₈ to C₃₆, particularly the C₁₁ to C₁₉ branched primary alcohol composition with or without ethylene oxide and/or propylene oxide, in which the number of ethoxy groups ranges from 3 to 12 and the ratio ethoxy/propoxy is from 4 to 12, followed by sulfation.

The general class of anionic surfactants or alcohol ethoxysulfates can be characterized by the chemical formula:

R'-O-(CH₂-CH₂-O) x -SO₃M (II)

wherein R' represents the branched olefin hydrophobe moiety, x represents the average number of oxyethylene groups per molecule and is in the range of from 0 to 12, and M is a cation selected from an alkali metal ion, an ammonium ion, and mixtures thereof. Of course, the surfactant can by oxyalkylated with any oxirane containing compound other than, in mixture with, or sequentially with ethylene oxide.

Sulfonation processes are described, for instance, in U.S. Patent Nos. 3,462,525, issued Aug. 19, 1969 to Levinsky et. al., 3,428,654 issued Feb. 18, 1969 to Rubinfeld-et. al., 3,420,875 issued Jan. 7, 1969 to DiSalvo et. al., 3,506,580 issued Apr. 14, 1970 to Rubinfeld et. al., 3,579,537 issued May 18, 1971 to Rubinfeld et. al., and 3,524,864 issued Aug. 18, 1970 to Rubinfeld. Suitable sulfation procedures include sulphur trioxide (SO₃) sulfation, chlorosulfonic acid (ClSO₃H) sulfation and sulfamic acid (NH₂SO₃H) sulfation. When concentrated sulfuric acid is used to sulfate alcohols, the concentrated sulfuric acid is typically from 75 to 100, preferably from 85 to 98 percent by weight, in water. Suitable amounts of sulfuric acid are generally in the range of from 0.3 to 1.3, preferably from 0.4 to 1.0 mole of sulfuric acid per mole of alcohol.

A typical sulphur trioxide sulfation procedure includes-contacting liquid alcohol or its ethoxylate and gaseous sulphur trioxide at about atmospheric pressure in the reaction zone of a falling film sulfator cooled by water at a temperature in the range of from 25 °C to 70 °C to yield the sulfuric acid ester of alcohol or its ethoxylate. The sulfuric acid ester of the alcohol or its ethoxylate then exits the falling film column and is neutralized with an alkali metal solution, e.g., sodium or potassium hydroxide, to form the alcohol sulfate salt or the alcohol ethoxysulfate salt.

Suitable oxyalkylated alcohols can be prepared by adding to the alcohol or mixture of alcohols to be oxyalkylated a calculated amount, e.g., from 0.1 to 0.6, preferably from 0.1 to 0.4 percent by weight, based on total alcohol, of a strong base, typically an alkali metal or alkaline earth metal hydroxide such as sodium hydroxide or potassium hydroxide, which serves as a catalyst for oxlyalkylation. The resulting mixture is dried, as by vapour phase removal of any water present, and an amount of alkylene oxide calculated to provide from 1 mole to 12 moles of alkylene oxide per mole of alcohol is then introduced and the resulting mixture is allowed to react until the alkylene oxide is consumed, the course of the reaction being followed by the decrease in reaction pressure.

The oxyalkylation is typically conducted at elevated temperatures and pressures. Suitable reaction temperatures range from 120 °C to 220 °C with the range of from 140 °C to 160 °C being preferred. A suitable reaction pressure is achieved by introducing to the reaction vessel the required amount of alkylene oxide which has a high vapour pressure at the desired reaction temperature. For consideration of process safety, the partial pressure of the alkylene oxide reactant is preferably limited, for instance, to less than 512 kPa, and/or the reactant is preferably diluted with an inert gas such as nitrogen, for instance, to a vapour phase concentration of about 50 percent or less. The reaction can, however, be safely accomplished at greater alkylene oxide concentration, greater total pressure and greater partial pressure of alkyelene oxide if suitable precautions, known to the art, are taken to manage the risks of explosion. With respect to ethylene oxide, a total pressure of between 376 and 858 kPa, with an ethylene oxide partial pressure between 345 and 621 kPa, is particularly preferred, while a total pressure of between 50 and 90 psig, with an ethylene oxide partial pressure between 238 and 445 kPa, is considered more preferred. The pressure serves as a measure of the degree of the reaction and the reaction is considered to be substantially complete when the pressure no longer decreases with time.

It should be understood that the oxyalkylation procedure serves to introduce a desired average number of alkylene oxide units per mole of alcohol oxyalkylate. For example, treatment of an alcohol mixture with 3 moles of ethylene oxide per mole of alcohol serves to effect the ethoxylation of each alcohol molecule with an average of 3 ethylene oxide moieties per mole alcohol moiety, although a substantial proportion of alcohol moieties will become combined with more than 3 ethylene.oxide moieties and an approximately equal proportion will have become combined with less than 3. In a typical ethoxylation product mixture, there is also a minor proportion of unreacted alcohol.

Other alkyene oxides can be used, such a proplyene oxide and butylene oxide. These may be added as a heteric mixture to the alcohol or sequentially to make a block stucture.

The sulfated primary alcohol composition of the invention can be used as surfactants in a wide variety of applications, including detergents such as granular laundry detergents, liquid laundry detergents, liquid dishwashing detergents; and in miscellaneous formulations such as general purpose cleaning agents, liquid soaps, shampoos and liquid scouring agents.

The sulfated primary alcohol composition of the invention find particular use in detergents, specifically laundry detergents. These are generally comprised of a number of components, besides the sulfated primary alcohol composition of the invention:
other surfactants of the ionic, nonionic, amphoteric or cationic type,
builders (phosphates, zeolites),cobuilders (polycarboxylates),
bleaching agents and their activators,
foam controlling agents,
enzymes,
anti-greying agents,
optical brighteners, and
stabilizers.

Liquid laundry detergents generally comprise the same components as granular laundry detergents, but generally contain less of the inorganic builder component. Hydrotropes are often present in the liquid detergent formulations. General purpose cleaning agents may comprise other surfactants, builders, foam suppressing agents, hydrotropes and solubilizer alcohols.

In addition to surfactants, washing and cleaning agents may contain a large amount of builder salts in amounts up to 90% by weight, preferably between 5-35% by weight, to intensify the cleaning action. Examples of common inorganic builders are phosphates, polyphosphates, alkali metal carbonates, silicates and sulfates. Examples of organic builders are polycarboxylates, aminocarboxylates such as ethylenediaminotetraacetates, nitrilotriacetates, hydroxycarboxylates, citrates, succinates and substituted and unsubstituted alkanedi- and polycarboxylic acids. Another type of builder, useful in granular laundry and built liquid laundry agents, includes various substantially water-insoluble materials which are capable of reducing the water hardness e.g. by ion exchange processes. In prarticular the complex sodium aluminosilicates, known as type A zeolites, are very useful for this purpose.

The formulations may also contain percompounds with a bleaching action, such as perborates, percarbonates, persulfates and organic peroxy acids. Formulations containing percompounds may also contain stabilizing agents, such as magnesium silicate, sodium ethylenediaminetetraacetate or sodium salts of phosphonic acids. In addition, bleach activators can be used to increase the efficiency of the inorganic persalts at lower washing temperatures. Particularly useful for this-purpose are substituted carboxylic acid amides, e.g., tetraacetylethylenediamine., substituted carboxylic acids, e.g., isononyloxybenzenesulfonate and sodiumcyanamide.

Examples of suitable hydrotropic substances are alkali metal salts of benzene, toluene and xylene sulfonic acids; alkali metal salts of formic acid, citric and succinic acid, alkali metal chlorides, urea, mono-, di-, and triethanolamine. Examples of solubilizer alcohols are ethanol, isopropanol, mono- or polyethylene glycols, monopropylene glycol and etheralcohols.

Examples of foam control agents are high molecular weight fatty acid soaps, paraffinic hydrocarbons, and silicon containing defoamers. In particular hydrophobic silica particles are efficient foam control agents in these laundry detergent formulations.

Examples of known enzymes which are effective in laundry detergent agents are protease, amylase and lipase. Preference is given to the enzymes which have their optimum performance at the design conditions of the washing and cleaning agent.

A large number of fluorescent whiteners are described in the literature. For laundry washing formulations, the derivatives of diaminostilbene disulfonates and substituted distyryIbiphenyl are particularly suitable.

As antigreying agents, water soluble colloids of an organic nature are preferably used. Examples are water soluble polyanionic polymers such as polymers and copolymers of acrylic and maleic acid, cellulose derivatives such as carboxymethyl cellulose methyl- and hydroxyethylcellulose.

In addition to one or more of the aforementioned other surfactants and other detergent composition components, compositions according to the invention typically comprise one or more inert components. For instance, the balance of liquid detergent composition is typically an inert solvent or diluent, most commonly water. Powdered or granular detergent compositions typically contain quantities of inert filler or carrier materials.

The following examples will illustrate the nature of the invention.

### Example 1

This example will demonstrate the manufacture of a skeletally isomerized C₁₆ olefin, subsequently converted to a skeletally isomerized C₁₇ primary alcohol composition according to the invention.

1 Litre of NEODENE 16 olefin, a C₁₆ linear α-olefin commercially available from Shell Chemical Company, was first dried and purified through alumina. The olefin was then passed through a tube furnace at about 250 °C set at a feed rate of about 1.0 ml/minute and using a nitrogen pad flowing at about 91 ml/minute. Working from the top, the tube furnace was loaded with glass wool, then 10 ml of silicon carbide, then the catalyst, followed by 5 ml of silicon carbide, and more glass wool at the bottom. The volume of the tube furnace was 66 ml. The reactor tube furnace had three temperature zones, with a multipoint thermocouple inserted into the tube reactor and positioned such that the temperature above, below and at three different places in the catalyst bed could be monitored. The reactor was inverted and installed in the furnace. All three zones, including the catalyst zone, were kept at about 250 °C during the reaction and the pressure was maintained in the reactor at 114 kPa.

The amount of catalyst used was 23.1g, or 53 ml by volume. The type of catalyst used to structurally isomerize the NEODENE 16 olefin was a 1.59 mm extruded and calcined H-ferrierite containing 100 ppm palladium metal.

This catalyst was prepared in accordance with example C of USP 5,510,306, reproduced in part herein for convenience. An ammonium-ferrierite having a molar silica to alumina ratio of 62:1, a surface area of 369 square meters per gram (P/Po = 0.03), a soda content of 480 ppm and n-hexane sorption capacity of 7.3 g per 100 g of zeolite was used as the starting zeolite. The catalyst components were mulled using a Lancaster mix muller. The mulled catalyst material was extruded using an 25.4 mm or a 57.2 mm Bonnot pin barrel extruder.

The catalyst was prepared using 1 wt% acetic acid and 1 wt% citric acid. The Lancaster mix muller was loaded with 645 grams of ammonium-ferrierite (5.4% Loss on Ignition) and 91 grams of CATAPAL D alumina (LOI of 25.7%). The alumina was blended with the ferrierite for 5 minutes during which time 152 millilitres of de-ionized water was added. A mixture of 6.8 grams glacial acetic acid, 7.0 grams of citric acid and 152 milliliters of deionized water was added slowly to the muller in order to peptize the alumina. The mixture was mulled for 10 minutes. 0.20 Grams of tetraammine palladium nitrate in 153 grams of de-ionized water were then-added slowly as the mixture was mulled for a period of 5 additional minutes. Ten grams of METHOCEL F4M hydroxypropyl methylcellulose was added and the zeolite/alumina mixture was mulled for 15 additional minutes. The extrusion mix had an LOI of 43.5%. The 90:10 zeolite/alumina mixture was transferred to the 2.25 inch Bonnot extruder and extruded using a die plate with 1.59 mm holes.

The moist extrudates were tray dried in an oven heated to 150 °C for 2 hours, and then increased to 175 °C for 4 hours. After drying, the extrudates were longsbroken manually. The extrudates were calcined in flowing air at 500 °C for two hours.

The olefin was passed through the reactor furnace over a 5 hour period. Samples of 36.99 g and 185.38 g were collected at about the 1 and 5 hour point, and combined for a total of about 222 g. A portion of this sample was then vacuum distilled at 0.533 kPa to obtain a predominate amount of the C₁₆ skeletally isomerized olefin by collecting distillate cuts boiling at 160 °C in the pot and 85 °C at the head, and 182 °C in the pot and 75 °C at the head.

A 90 Gram sample of the 110.93 grams of the skeletally isomerized olefin was then hydroformlyated using the modified oxo process. 90 Grams of the skeletally isomerized olefin was reacted with hydrogen and carbon monoxide in about a 1.7:1 molar ratio in the presence of a phosphine modified cobalt catalyst at a temperature of up to about 185 °C and a pressure of about 7684 kPa for four and one-half hours in a nitrogen purged 300cc autoclave. After completion of the reaction, the product was cooled to 60 °C.

40 Grams of the hydroformylated product was poured into a 100 ml flask and vacuum distilled for 4 hours at 0.533 kPa with temperature increases from start of 89 °C to a finish temperature of 165 °C. Distillate cuts of 20.14 g and 4.12 g were taken at 155 °C and 165 °C, respectively, and combined in a 100 ml flask.

To the distillate cuts in the flask was added 0.2 g of sodium borohydride, stirred, and heated up to 90 °C over an 8 hour period to deactivate the hydroformylation catalyst and stabilize the alcohols. The distilled alcohol was washed with 90 °C water three times, dried with sodium sulfate, and filtered into a 100 ml flask. The alcohol was then vacuum distilled for a further hour to distill off any remaining water. The product was then subjected to NMR analysis and sulfation to test for cold water solubility, detergency, and biodegradability.

### Example 2

This example will demonstrate the manufacture of a skeletally isomerized C₁₃₋₁₄ olefin, subsequently converted to a skeletally isomerized C₁₄₋₁₅ primary alcohol composition according to the invention.

A C13-14 linear internal olefin having a composition of 53.38% linear C13 olefin, 45.24% of linear C14 olefin, 0.96% branched C13 olefin, and 0.29% branched C14 olefin, was subjected to skeletal isomerization using the same procedure and type of equipment as described above in example 1. The olefin was passed through the tube furnace for 26 hours, except that after 8 hours the temperature of the tube furnace was increased in all three zones to 275 °C. At the 13 hours, 18 hours, 20 hours, and 26 hours mark, samples of the skeletally isomerized olefins were collected and combined for a total of 774 g.

Samples of gaseous and liquid products collected after 4.5 hours (at 250 °C) and 15.5 hours (at 275 °C) on stream were analyzed in order to determine their composition. At 250 °C, 70.1% of the C13 olefin feed was converted, and 75.6% of the C14 olefin feed-was converted. At these conversion levels, the selectivity to branched C13 and C14 olefins was 96.3% and 97.8%, respectively. 67.4% of the C13 olefin feed was recovered as skeletally isomerized C13 branched olefin. 74.0% of the C14 olefin feed was recovered as skeletally isomerized C14 branched olefin.

At 275 °C, 79.4% of the C13 olefin feed was converted, and 82.2% of the C14 olefin feed was converted. At these conversion levels, the selectivity to branched C13 and C14 olefins was 91.5% and 92.1%, respectively. 72.6% of the C13 olefin feed was recovered as skeletally isomerized C13 branched olefin. 75.6% of the C14 olefin feed was recovered as skeletally isomerized C14 branched olefin.

The skeletally isomerized olefin was then vacuum distilled at 0.533 kPa. 636 Gram of distillate boiling in the pot at temperatures in the range of 135 °C to 145 °C and at the head within the range of 108 °C to 138 °C were collected.

606 Gram of the skeletally isomerized distilled olefin was hydroformylated by the above procedure, except in a 3.785 litre autoclave using a 37/63 mole% ratio of carbon monoxide to hydrogen for a period of 12-13 hours at 4826 to 5516 kPa and 175 °C. 693 Gram of alcohol was collected.

The alcohol was then flash distilled at 0.533 kPa to collect the C₁₄₋₁₅ alcohol, with about 650 g of distillate cut boiling in pot at 185 °C and at the head at 140 °C collected. This cut was treated with 5.0 g of sodium borohydride, heated to about 100 °C, and then treated with 5.0 more grams of sodium borohydride, for a total heat time of 9 hours. The alcohol was washed with 90 °C water three times, dried with sodium sulfate, filtered, and vacuum distilled at 0.533 kPa. Distillate cuts boiling at 128 °C through 142 °C at the head were collected and tested with NMR, after which they were sulfated and tested for cold water solubility, detergency, and biodegradability.

### Example 3

The same procedure as used in example 1 was used to skeletally isomerize a NEODENE 14 olefin commercially available from Shell Chemical Company, which is a C₁₄ α-olefin, with subsequent conversion to a skeletally isomerized C₁₅ primary alcohol composition. The tube furnace was kept at 250 °C. The skeletally isomerized distillate cut boiling at 133 °C in the pot and 64 °C at the head was collected and hydroformylated at 8963-9653 kPa for 5 hours at a molar ratio of H₂/CO of 1.7:1, using the equipment of example 1.

### Example 4

The same procedure as used in example 1 was employed to skeletally isomerize a NEODENE 12 olefin, a C₁₂ α-olefin, subsequently converted to a skeletally isomerized C₁₃ primary alcohol composition. The skeletally isomerized olefin was vacuum distilled at 2.665 kPa, and the distillate cut boiling at 172 °C in the pot and 105 °C at the head was collected and hydroformylated to an alcohol. The hydroformylation equipment was as used in example 2, at 2032 kPa over an 8 hour period, using a 37/63 mole% CO/H gas mixture. The alcohol was vacuum distilled at 1.333 kPa, with those cuts boiling at 141-152 °C in the pot and 127-132 °C at the head being collected.

### Example 5

The same olefin, procedure, and type of equipment as used in example 2 was repeated. The C₁₃₋₁₄ internal olefin was skeletally isomerized at 250 °C. The isomerized olefin was vacuum distilled at 0.533 kPa, with distillate cuts boiling at 95 °C and 77 °C at the head being collected, as well as distillate cuts boiling between 120 °C to 175 °C in the pot and 73 °C to 106 °C at the head being collected under 2.665 kPa. The hydroformylation was conducted in an autoclave for about 9 hours at a pressure of 8032 kPa using a CO to H gas ratio of 37/63 mole%. Afterwards, the distillate cut boiling at 173 °C in the pot and 125 °C at the head was collected and treated with sodium borohydride as in example 2.

### Example 6

Each of the primary alcohol compositions described in examples 1-6 were sulfated by adding dropwise chlorosulfonic acid to the primary alcohol composition. Specifically, the primary alcohol composition was sparged for 2-3 hours with nitrogen in a flask, after which about 1 ml of methylene chloride per gram of the primary alcohol composition was added. The chlorosulfonic acid was added dropwise to the primary alcohol composition in the flask for about 25 minutes, while maintaining the temperature at about 30-35 °C. More methylene chloride was added if the solution became to viscous. The solution was then sparged with nitrogen for 2-3 minutes to facilitated removal of HCl, after which it was added slowly to a chilled 50% sodium hydroxide in 3A alcohol solution to neutralize the primary alcohol composition. If the pH was below 8, more of the basic solution was added, until the pH was adjusted to between 8-9. If too acidic, a 50% solution of H₂SO₄ was added to adjust the pH. The solution was stirred for another hour, and the pH adjusted accordingly within the stated range. Methylene chloride was removed by a rotary evaporator under reduced pressure at about 40 °C under a nitrogen sparge.

The primary alcohol compositions were subsequently tested for amount, type, and location of branching using the JSME NMR method described herein. For a determination of quaternary carbon atoms, the quat only JSME NMR technique described herein was used. These results are reported in Table 1 below. The sulfated primary alcohol samples were also tested for biodegradability, the results of which are reported in Table II; and detergency, the results of which are reported in Table III. The examples reported in the tables are arranged by order of chain length for ease of viewing, and identified as 6- indicating the sulfate of a corresponding example number. Each of these tests were conducted in accordance with the procedures specified above. As a comparison example, NEODOL 45-Sulfate was tested for branching, biodegradability, and detergency. NEODOL 45-S was used as the comparison because it is the current commercial primary alcohol composition, which when sulfated, is currently used in detergents and is known for its ready biodegradability.

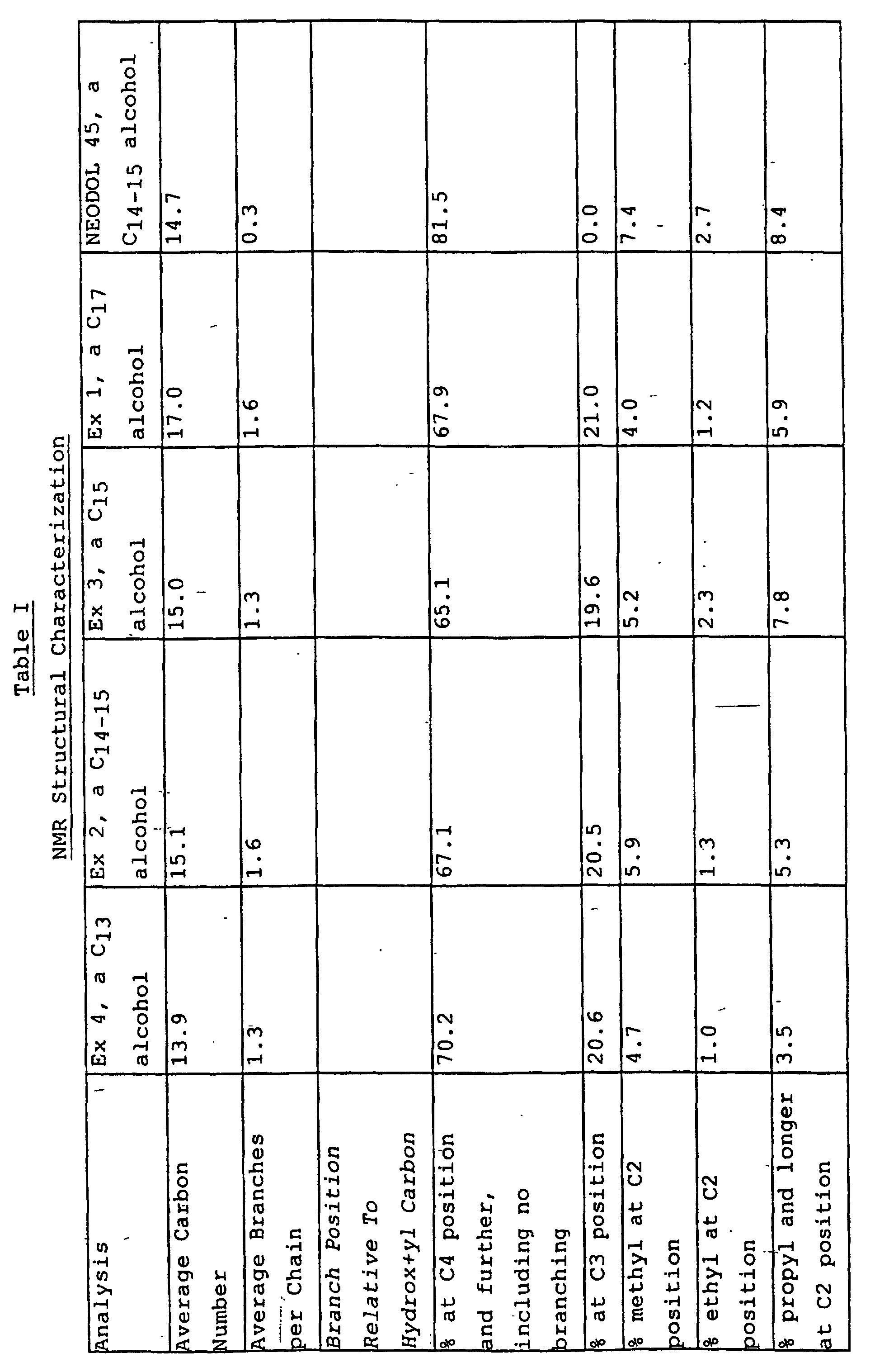

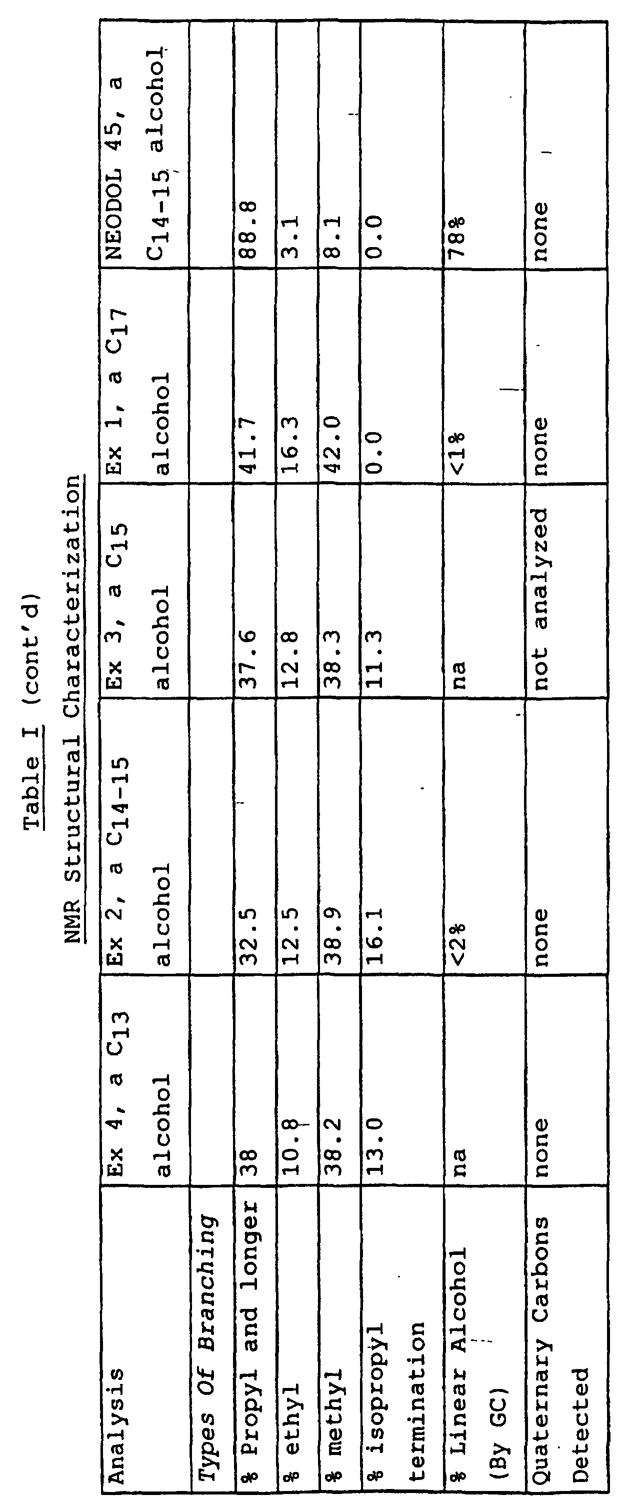

The results above indicate that the skeletally isomerized branched alcohols according to the invention have a very high average number of branches per molecule chain, well exceeding 0.7, while the commercial NEODOL 45 has an average number of branches which is quite low, on the order of 0.3. The patterns of branching are strikingly similar for the different alcohols according to the invention except that the branched C₁₇ is curiously deficient in isopropyl termination. The results also indicate a sharp increase in the number of branches occurring at the C₃ position compared to the lack of any branches in the NEODOL 45 alcohol at the C₃ position. Of the types of branches detected, most of the branches are methyl groups for both the skeletally isomerized alcohols and the NEODOL alcohol. However, the-skeletally isomerized alcohol methyl branches are not concentrated at the C₂ position, as is the case for NEODOL 45 and other conventional detergent range alcohols. A further distinguishing feature of the skeletally isomerized alcohols is that they contain a larger proportion of ethyl types of branches than the NEODOL 45. Further, except the C₁₇ branched alcohol, most of the embodiments were also skeletally isomerized at the terminal part of the hydrophobe, as indicated by the high percentage of terminal isopropyl formation, in contrast to none found in the NEODOL 45.

The results also support a conclusions that a predominate number of branches in the skeletally isomerized alcohols are concentrated towards the ends of the molecule chain, i.e., at the C₂, C₃, and at the isopropyl terminal position, rather than towards the center of the molecule chain. NMR data showing a high percentage of methyl, ethyl, and isopropyl branching for a compound whose branching is predominately towards the center of the chain, i.e. inward from the fourth carbon on either end of the chain, typically have very low percentages of branching at the C₂ and C₃ positions. The data above, however, shows both a high percentage of methyl, ethyl, and isopropyl types of branches as well as a high amount of branching occurring at the C₂ and C₃ positions, indicating that the molecule has a higher concentration of branches at the C₂ and C₃ carbon positions at the ends of the carbon chain than the number of branches found at the C₄ or longer positions from both ends of the molecule proceeding inward towards the center.

Finally, in spite of the high number of branches per molecule chain, no quaternary carbon atoms were detected by the modified NMR JSME method. This would suggest that these compounds should readily biodegrade.

**Table II**

| % Biodegradation of Skeletally Isomerized Alcohol Sulfates | | | | |
|---|---|---|---|---|
| Example No. | 5-day | 10-day | 15-day | 28-day |
| 6-4, a C₁₃ alcohol sulfate | 47 | 61 | 71 | 100 |
| 6-2, a C₁₄₋₁₅ alcohol sulfate | 38 | 58 | 65 | 100 |
| 6-3, a C₁₅ alcohol sulfate | 22 | 48 | 63 | 69 |
| 6-1, a C₁₇ alcohol sulfate | 44 | 56 | 70 | 89 |
| A sulfated NEODOL C₁₄₋₁₅ alcohol | 44 | 63 | 78 | 86 |

The OECD 301D biodegradation results indicate that each of the sulfated primary alcohol compositions of the invention readily biodegraded. Some of the sulfated primary alcohol compositions of the invention even exhibited 100% biodegradation at 28 days.

**Table III**

| Multisebum Detergencies of Skeletally Isomerized Alcohol Sulfates | | |
|---|---|---|
| Example No. | 10 °C | 32 °C |
| 6-4, a C₁₃ alcohol sulfate | 12 | 14 |
| 6-2, a C₁₄₋₁₅ alcohol sulfate | 37 | 49 |
| 6-3, a C₁₅ alcohol sulfate | 39 | 50 |
| 6-1, a C₁₇ alcohol sulfate | 24 | 35 |
| A sulfated NEODOL C₁₄₋₁₅ alcohol | 16 | 34 |

LSD₉₅ (Least Significant Difference at 95% probability) is 5.0 at both temperatures.

The detergency results indicate that the alcohol sulfate compositions of the invention exhibited extremely good cold water detergency. For example, 6-2 far out-performed the sulfated NEODOL alcohol, each of equal chain length, in both cold and warm water detergency. A composition having good cold water detergency is one in which has superior cold water detergency over a sulfated NEODOL alcohol of the same chain length. Preferred, however, are those alcohol sulfates which have a cold water detergency of 22% or more, most preferably 28% or more.

### Example 7

This example demonstrates the manufacture of a dimerized monobranched C₁₂-C₁₅ alcohol from internal olefins using a nickel chelate catalyst.

A flask was charged with 2268.7 grams of a composition of C₆-C₈ internal olefins containing some 4, 5, 9, and 10 carbon olefins, and distilled using an 11 plate Oldershaw distillation column with a swinging bucket reflux splitter condensor, a dry ice chilled trap, and a nitrogen blanket. After 37 hours of distillation, those cuts distilling up to 138 °C in the pot and 125 °C at the head were collected for a total amount of about 1200 grams. These cuts represent the light ends of the olefin, C₄₋₈.

The 1200 grams of the C₄₋₈ olefin feed was dimerized by the following method. The 1200 grams of the olefin was poured into a 5 litre round bottom flask equipped with a condensor, a dry ice condensor, a thermocouple, a water bath, and a nitrogen blanket. 19.74 Gram of dried nickel hexafluoroacetoacetyl acetonate (nickel catalyst) and 53.76 g of an 11/89 wt.% solution of diethylaluminium ethoxide in cyclohexane (aluminium solution) were added sequentially and stirred into the olefin. The reaction mixture was heated up to 35 °C for 6.5 hours, after which 14.38 more grams of the aluminium solution were added, heated up to 37 °C for an additional 2 hours, after which 4.0 more grams of the nickel catalyst and 13.75 g of the aluminium solution were added, heated up to 35 °C to 37 °C for another 10 hours, after which 15.55 more grams of the aluminium solution were added followed by heating for another 4 hours, after which 4 more grams of the nickel catalyst and 14.4 more grams of the aluminium solution were added, followed by heating for another 5 hours, after which 21.0 more grams of the aluminium solution and 5.0 grams of the nickel catalyst were added, followed by heating for another 3 hours, after which 4.18 grams of the nickel catalyst and 20.1 more grams of the aluminium solution were added.

Subsequently, the reaction product in the flask was quenched with 100 g of citric acid and 22 g of a sodium bicarbonate solution per 0.946 litre (quart) of water, and filtered.

The dimerized C₄₋₈ olefin was then subjected to further distillation to obtain cuts having predominantly C₁₃₋₁₄ olefins. The distillation was conducted as above, except with a 10 plate Oddershaw column, and those cuts distilling at 169 °C to 194 °C in the pot and 129 °C to 155 °C at the head, at 6.3-6.4 kPa vacuum, were collected, for a total of 188.05 grams.

150 grams of this batch was then subjected to hydroformylation in a 500 ml autoclave, using the modified oxo process. The 150 grams of the dimerized olefin were reacted with hydrogen and carbon monoxide at a H₂/CO ratio of 2, in the presence of a phosphine modified cobalt catalyst and potassium hydroxide in ethanol at a temperature of up to 180 °C, a stirring speed of 1250 rpm, and a pressure of 6894 kPa, for 20 hours. After completion of the reaction, the product was cooled to 60 °C.

The hydroformylated dimerized alcohols were subjected to further flash volatilization do separate out any unconverted olefin and paraffins. Those cuts distilling at 182 °C to 250 °C in the pot and 99 °C to 112 °C at the head were collected, and neutralized with sodium borohydride. The distillate cuts, totaling 300 ml, were added to a round bottom flask, stirred and heated to 50 °C, to which 0.6 grams of the sodium borohydride were added and reacted for about 2 hours, after which 0.6 more grams of the sodium borohydride were added and reacted for another 1.5 hours at 75-80 °C, and then reacted for another 2 and 1/2 hours at 98-100 °C. The solution was left to cool, transferred to a 500 ml flask, washed by shaking with 70 °C deionized water under ventilation, let stand, to which was added 20 ml of ethyl ether, shaken, and separated. The water phase was drained and the process repeated another two times using ethyl ether. After washing, 10 grams of sodium sulfate was added to the alcohol, shook, and then let stand. The product was filtered, the liquid transferred to a 250 ml flask, and then subjected to further distillation to rid the solution of the light ends. The distillates obtained up to 102 °C in the pot and 163 °C at the head were discarded, and 82.91 ml of the contents in the pot were recovered. These contents were monobranched C₁₂₋₁₆ alcohols, having 42% C₁₄, 44% C₁₅, and 8% C₁₆ alcohols as determined by GCMS, and were subjected to analytical testing and further reaction for making the sulfates.

### Example 8

This example demonstrates the preparation of a C₁₃₋₁₇ dimerized monobranched alcohol from internal olefins using a nickel carboxylate catalyst.

The same procedure as used in Example 1 above was followed with the following noted exceptions. The amount of C₄₋₁₀ internal olefins distilled was 2427.7 grams. 712.5 grams of distillate boiling at 120 to 154 °C in the pot and 89 °C to 129 °C at the head were collected. The reflux was set at 5 seconds on, and 7 seconds off. The distillate cuts were predominantly C₆₋₉-carbon chain internal olefins. 702.6 grams of these olefins were dimerized in a 2 litre flask using 0.493 g of nickel 2-ethylhexanoate-tirfluoroacetate in 5 ml of cyclohexane and 12 ml of a 1 molar solution of ethylaluminium dichloride in hexane, (first batch of catalyst) as the dimerization catalysts injected into the olefin. The contents of the flask were heated to 35-38 °C on average throughout the course of the reaction. After about 3 hours of heat, a second batch catalyst in the same amount was added. After another hour of heat, a third batch of catalyst in the same amount was added, and after another hour and 15 minutes, a fourth batch of catalyst in the same amount was added. After 6.5 hours, the fifth batch of catalyst in the same amount was added, and after another 7 hours of heat, another catalyst batch in the same amount was added, and finally after another 1.5 hours, the final catalyst batch in the same amount was added. The contents of the flask were heated for another hour.

To neutralize the dimerization catalyst, 22 g of sodium bicarbonate in 250 g of deionized water was added to 100 g of citric acid in 100 grams of deionized water, to which was added more water to make a 1 litre batch. The dimerized olefins were poured into a 2 litre separation funnel with 1/2 litre of the citric acid/bicarbonate solution, shook and vented, separated, and repeated. The neutralized dimerized solution was dried with sodium sulfate as above.

As in example 1, the olefins were further distilled to acquire C₆₋₉ olefins. Those distillate cuts boiling at 157 °C in the pot and 125 °C at the head at 5.5 kPa, and those boiling at 139 °C to 164 °C in the pot at 1.866 kPa, and those boiling at from 179 °C to 240 °C in the pot at 1.9 kPa were collected for a total of 231.95 grams of distillate.

The dimerized distillate was hydroformylated as above and flash distilled at about 0.5-0.7 kPa. 1.39 Gram of sodium borohydride was added to 211.48 g of the distilled alcohol, heated to 50 °C for 1 hour, after which another 1.3 g of sodium borohydride was added and heated to 90 °C for four hours and cooled.

The product was washed as above, and redistilled with those distillate cuts boiling at 141.5 to 240 °C in the pot and 100 °C to 104 °C at the head being collected at 0.4 kPa. The monobranched C₁₃₋₁₇ alcohols, having 25% C₁₄, 40% C₁₅, and 25% C₁₆ alcohols, as determined by GCMS, were collected and subjected to analytical testing and sulfation as described below.

### Example 9

This example demonstrates the preparation of a C_{13,15,17} dimerized monobranched alcohols from alpha olefins.

In this example, a mixture of 600g NEODENE 6 α-olefin, a C₆ olefin, and 800 g of NEODENE 8 α-olefin, a C₈ olefin, containing 5.32 g of ethylaluminium dichloride, were added to a 5 litre flask. The same procedure as used in example 1 was followed with the following differences. A solution of 7.9 g of the nickel 2-ethylhexanoate-tirfluoroacetate in 6.35 g of cyclohexane (the nickel solution) was added and heated.- The flask was maintained at from 33 °C to 38 °C throughout the course of the reaction. Another 7.6 ml of the aluminium solution as prepared in example 2 and 5 ml of the nickel solution were injected into the reaction flask after about 8 hours of heating.

1.5 Litres of the sodium citrate neutralizing solution was used to neutralize the dimerized olefins, separated, and again repeated. The dimerized product was distilled, with the cuts distilling at 149 °C to 160 °C in the pot and 137 °C to 148 °C at the head, at 8.0 kPa, 120 °C to 133 °C in the pot and 110 to 122 °C at the head at 1.2 kPa, and 127 to 149 °C in the pot and 118 °C to 145 °C at the head at 1.3 kPa being collected for a total of 786.4 g.

730 G of these dimerized olefins were hydroformylated in a 3.785 litre autoclave, reacted at temperatures up to 240 °C at pressures up to 7894 kPa.

809 G of the hydroformylated olefins were treated with 6.5 g of sodium borohydride, as above, followed by another addition of 6.5 g of sodium borohydride and heating, and a third addition of 4.95 g followed by 6 hours of heating at up to 99 °C.

The treated hydroformylated olefins were washed as in example 1, filtered, and distilled with those cuts distilling at 152 to 181 °C in the pot and 137 to 172 °C at the head at 0.8 kPa being collected for a total of 495 g of C₁₃, C₁₅, and C₁₇ monobranched alcohols. The sample was analytically tested and sulfated as described below.

### Example 10

Each of the monobranched alcohol compositions described in examples 7-9 were sulfated by adding dropwise chlorosulfonic acid to the alcohol composition. Specifically, the alcohol compositions were sparged for 2-3 hours with nitrogen in a flask, after which about 1 ml of methylene chloride per gram of the alcohol composition was added. The chlorosulfonic acid was added dropwise to the alcohol composition in the flask for about 25 minutes, while maintaining the temperature at 30-35 °C. More methylene chloride was added if the solution became to viscous. The solution was then sparged with nitrogen for 2-3 minutes to facilitate removal of HCl, after which it was added slowly to a chilled 50% sodium hydroxide in 3A alcohol solution to neutralize the alcohol composition. If the pH was below 8, more of the basic solution was added, until the pH was adjusted to between 8-9. If too acidic, a 50% solution of H₂SO₄ was added to adjust the pH. The solution was stirred for another hour, and the pH adjusted accordingly within the stated range. Methylene chloride was removed by a rotary evaporator under reduced pressure at 40 °C under a nitrogen sparge.

The alcohol compositions of examples 1-3 were subsequently tested for amount, type, and location of branching using the JSME NMR method described herein. For a determination of quaternary carbon atoms, the quat only JSME NMR technique described herein was used. These results are reported in Table 1 below. The average carbon number was determined by GCMS. The sulfated primary alcohol samples were also tested for biodegradability, the results of which are reported in Table II; and detergency, the results of which are reported in Table III. The examples reported in the tables are arranged by order of chain length for ease of viewing, and identified as 10- indicating the sulfate of a corresponding example number. Each of these tests were conducted in accordance with the procedures specified above. As a comparison example, NEODOL 45-Sulfate was tested for branching, biodegradability, and detergency. NEODOL 45-S was used as the comparison because it is the current commercial primary alcohol composition, which when sulfated, is currently used in detergents and is known for its ready biodegradability. Also as a comparison, a sulfated EXXAL-13S alcohol believed to have a predominance of C₁₃ alcohols and derived from propylene oligomerization through acid catalysis and then subjected to hydroformylation using an oxo process, was subjected to biodegradation testing. EXXAL 13 is reported to have about 3-4 methyl branches per tridecyl-alcohol molecule.

**Table IV**

| NMR Structural Characterization | | | | |
|---|---|---|---|---|
| Analysis | Ex 7, a C₁₂₋₁₅ alcohol | Ex 8, a C₁₃₋₁₅ alcohol* | Ex 9, a C_{13,15,17} alcohol | NEODOL 45, a C₁₄₋₁₅ alcohol |
| Average Carbon Number | 14.5 | 15.5 | 16.8 | 14.7 |
| Average Branches per Chain | 1.0 | 1.4 | 1.5 | 0.3 |
| *Branch Position Relative To Hydroxyl Carbon* | | | | |
| %@ C4 position and further, including no branching | 83.7 | 85 | 83.4 | 81.5 |
| %@ C3 position | 4.7 | 3 | 2.7 | 0.0 |
| % methyl @ C2 position | 6.4 | 6 | 7.4 | 7.4 |
| % ethyl @ C2 position | 1.6 | 2 | 6 | 2.7 |
| % propyl and longer @ C2 position | 3.6 | 4 | 4.1 | 8.4 |
| *Types Of Branching* | | | | |
| % Butyl or longer | 52.2 | 28 | | |
| % Propyl | 17.5 | 12 | 66.7** | 88.8** |
| % ethyl | 16 | 23 | 12.9 | 3.1 |
| % methyl | 14.2 | 37 | 20.4 | 8.1 |
| Quaternary Carbons Detected | na | none | na | none |

| | | | | |
|---|---|---|---|---|
| * Approximately 21% of the branching was conjugated with methyl branches on adjacent carbons in the chain. | | | | |
| **Includes propyl and butyl branching. | | | | |

The results indicate that the dimerized alcohols according to the invention look very much the same as NEODOL alcohols with respect to the branch positions, according to the NMR analysis. Specifically, very few branches are located at the C₂₋₄ carbon positions. Since the average number of branches of the dimerized alcohols far exceeds that of NEODOL alcohols, the center of the molecule carbon backbone must be where the predominant number of branches are situated, i.e. an excess of 80%. By center is meant the C₄ position inward from each end of the molecule.

Also noteworthy is the higher percentage of ethyl branches on the dimerized alcohols of the invention as compared to the relatively few ethyl branches found in the NEODOL alcohol.

**Table V**

| % Biodegradation of Dimerized Alcohol Sulfates | | | | |
|---|---|---|---|---|
| Example No. | 5-day | 10-day | 15-day | 28-day |
| 10-7 | 19 | 43 | 61 | 68 |
| 10-8 | 23 | 39 | 65 | 73 |
| 10-9 | 28 | 35 | 60 | 64 |
| A sulfated NEODOL C₁₄₋₁₅ alcohol | 40 | 64 | 71 | 75 |
| A sulfated EXXAL 13-S | 4 | 12 | 21 | 40 |

The OECD 301D biodegradation results indicate that each of the sulfated primary alcohol compositions of the invention biodegraded readily, as did the NEODOL sulfated alcohol. The sulfated EXXAL alcohol biodegraded only poorly.

**Table VI**

| Multisebum Detergencies of Dimerized Alcohol_Sulfates | | |
|---|---|---|
| Example No. | 10 °C | 32 °C |
| 10-7 | 27 | 31 |
| 10-8 | 21 | 31 |
| 10-9 | 15 | 25 |
| A sulfated NEODOL C₁₄₋₁₅ alcohol | 11 | 29 |

LSD₉₅ (Least Significant Difference at 95% probability) is 5.0 at both temperatures.

The detergency evaluations indicate that the dimerized alcohols of the invention have superior or equal cold water detergency as compared to the conventional NEODOL sulfated alcohol.

## Claims

1. A branched primary alcohol composition, having from 11 to 36 carbon atoms and an average number of branches per molecule of from 0.7 to 3.0, said branching comprising methyl and ethyl branches, said composition comprising less than 0.5 atom % of quaternary carbon atoms.

2. A branched primary alcohol according to claim 1, wherein the average number of branches per molecule ranges from 1.0 to 3.0.

3. A branched primary alcohol composition according to claim 1 or 2, wherein the average number of branches per molecule ranges from 1.5 to 2.3.

4. A branched primary alcohol composition according to any one of claims 1 to 3, containing less than 5% of linear alcohols.

5. A branched composition according to any one of claims 1 to 4, wherein at least 40% of the number of branches in the alcohol are methyl branches.

6. A branched alcohol composition according to any one of claims 1 to 5, wherein from 5% to 30% of the number of branches in the alcohol are ethyl branches.

7. A branched primary alcohol composition according to any one of claims 1 to 6, having from 5 to 25% branching on the C₂ carbon position, relative to the hydroxyl carbon atom.

8. A branched primary alcohol composition according to any one of claims 1 to 6, wherein from 10 to 20% of the number of branches are at the C₂ position.

9. A branched primary alcohol composition according to any one of claims 1 to 8, wherein said composition has from 10 to 50% of the number of branches on the C₃ position.

10. A branched primary alcohol composition according to claim 9, wherein said composition has from 15 to 30% of the number of branches on the C₃ position.

11. A branched primary alcohol composition according to any one of claims 1 to 10, wherein said composition has at least 5% of isopropyl terminal type of branching.

12. A process for preparing a branched primary alcohol composition according to claim 1, which process comprises the steps of:
a) contacting an olefin feed comprising a linear olefin having at least 10 carbon atoms with a catalyst effective for skeletally isomerising said linear olefin to yield a branched olefin of the same carbon number; and
b) converting said branched olefin to said primary alcohol composition.

13. A process according to claim 12, wherein the skeletally isomerising catalyst comprises a molecular sieve having at least one channel with a crystallographic free diameter along the x and/or y planes of the [001] view ranging from 0.42 to 0.70 nanometer, the molecular sieve preferably being a zeolite having the ferrierite isotypic structure.

14. A process for preparing a branched primary alcohol composition according to claim 1, having from 13 to 21 carbon atoms, which process comprises the steps of:
a) dimerising, in the presence of a homogeneous dimerisation catalyst, an olefin feed comprising a C₆-C₁₀ olefin to yield a C₁₂-C₂₀ branched olefin; and
b) Converting said C₁₂-C₂₀ branched olefin to said branched primary alcohol composition.

15. A process according to claim 14, wherein the olefin feed comprises at least 90% of linear olefins.

16. A process according to claim 14 or 15, wherein the olefin feed comprises at least 50% of internal olefins.

17. A process according to any one of claims 14 to 16, wherein said dimerisation catalyst comprises a combination of a nickel carboxylate,with an alkyl aluminium halide, or a combination of a nickel chelate with an alkyl aluminium alkoxide.

18. A process according to any one of claims 12 to 17, wherein the conversion of the olefin to the alcohol in step b) is effected by hydroformylating the olefin with carbon monoxide and hydrogen, in the presence of a hydroformylating catalyst.

19. A branched primary alcohol alkoxylate composition, preparable by reacting a branched primary alcohol composition according to any one of claims 1 to 11 with an oxirane compound.

20. A branched primary alcohol alkoxylate composition according to claim 19 wherein the alkoxylate is predominantly ethoxylate, preparable by reacting the primary alcohol composition with ethylene oxide.

21. A branched primary alkyl sulphate, preparable by sulfating a primary alcohol composition according to any one of claims 1 to 11.

22. A branched alkoxylated primary alkyl sulphate, preparable by alkoxylating and sulphating a branched primary alcohol composition according to any one of claims 1 to 11.

23. A branched primary alkyl carboxylate, preparable by oxidising a branched primary alcohol composition according to any one of claims 1 to 11.

24. A detergent composition comprising:
a) one or more surfactant(s) selected from the group of branched primary alcohol alkoxylates according to claim 19, branched primary alkyl sulphates according to claim 21, and branched alkoxylated primary alkyl sulphates according to claim 22;
b) a builder; and
c) optionally one or more additives selected from the group of foam controlling agents, enzymes, bleaching agents, bleach activators, optical brighteners, co-builders, hydrotropes and stabilisers.

25. A detergent composition according to claim 24, wherein the builder is chosen from the group of alkali metal carbonates, silicates, sulphates, polycarboxylates, aminocarboxylates, nitrilotriacetates, hydroxycarboxylates, citrates, succinates, substituted and unsubstituted alkane di- and poly-carboxylic acids, complex aluminosilicates, and mixtures thereof.

26. A detergent composition according to claim 24 or 25, containing a bleaching agent which is selected from the group of perborates, percarbonates, persulfates, organic peroxy acids, and mixtures thereof.

27. A detergent composition according to any one of claims 24 to 26, containing a bleach activator which is selected from the group of carboxylic acid amides, substituted carboxylic acids, and mixtures thereof.

28. A detergent composition according to any one of claims 24 to 27, containing a hydrotrope which is selected from the group of alkali metal salts of aromatic acids or alkyl carboxylic acids, alkali metal chlorides, urea, mono- or polyalkanolamines, and mixtures thereof.

29. A detergent composition according to any one of claims 24 to 28, which is chosen from the group of granular laundry detergents, liquid laundry detergents, dishwashing detergents, soaps, shampoos, and scouring detergents.

## Patentansprüche

1. Zusammensetzung auf der Basis verzweigter primärer Alkohole mit 11 bis 36 Kohlenstoffatomen und durchschnittlich 0,7 bis 3,0 Verzweigungen pro Molekül, welche Verzweigungen Methyl- und Ethylverzweigungen umfassen, welche Zusammensetzung weniger als 0,5 Atomprozent an quaternären Kohlenstoffatomen umfaßt.

2. Verzweigter primärer Alkohol nach Anspruch 1, worin die mittlere Anzahl von Verzweigungen pro Molekül von 1,0 bis 3,0 beträgt.

3. Zusammensetzung auf der Basis verzweigter primärer Alkohole nach Anspruch 1 oder 2, worin die mittlere Anzahl von Verzweigungen pro Molekül von 1,5 bis 2,3 beträgt.

4. Zusammensetzung auf der Basis verzweigter primärer Alkohole nach einem der Ansprüche 1 bis 3, mit einem Gehalt an weniger als 5% linearen Alkoholen.

5. Verzweigte Zusammensetzung nach einem der Ansprüche 1 bis 4, worin wenigstens 40% der Anzahl von Verzweigungen in dem Alkohol Methylverzweigungen sind.

6. Zusammensetzung auf der Basis verzweigter Alkohole nach einem der Ansprüche 1 bis 5, worin 5 bis 30% der Anzahl der Verzweigungen in dem Alkohol Ethylverzweigungen sind.

7. Zusammensetzung auf der Basis verzweigter primärer Alkohole nach einem der Ansprüche 1 bis 6, mit 5 bis 25% Verzweigungen an der C₂-Kohlenstoffposition, bezogen auf das Hydroxylkohlenstoffatom.

8. Zusammensetzung auf der Basis verzweigter primärer Alkohole nach einem der Ansprüche 1 bis 6, worin 10 bis 20% der Anzahl von Verzweigungen an der C₂-Position vorliegen.

9. Zusammensetzung auf der Basis verzweigter primärer Alkohole nach einem der Ansprüche 1 bis 8, worin die Zusammensetzung 10 bis 50% der Anzahl der Verzweigungen an der C₃-Position aufweist.

10. Zusammensetzung auf der Basis verzweigter primärer Alkohole nach Anspruch 9, worin die Zusammensetzung von 15 bis 30% der Anzahl von Verzweigungen an der C₃-Position aufweist.

11. Zusammensetzung auf der Basis verzweigter primärer Alkohole nach einem der Ansprüche 1 bis 10, worin die Zusammensetzung wenigstens 5% einer Verzweigung vom endständigen Isopropyltyp aufweist.

12. Verfahren zur Herstellung einer Zusammensetzung auf der Basis verzweigter primärer Alkohole nach Anspruch 1, welches Verfahren die folgenden Stufen umfaßt:
a) Inkontaktbringen eines Olefineinsatzmaterials, das ein lineares Olefin mit wenigstens 10 Kohlenstoffatomen umfaßt, mit einem zur Skelettisomerisierung dieses linearen Olefins wirksamen Katalysator zur Ausbildung eines verzweigten Olefins mit gleicher Kohlenstoffanzahl; und
b) Umwandeln dieses verzweigten Olefins in die Zusammensetzung auf Basis primärer Alkohole.

13. Verfahren nach Anspruch 12, worin der Skelettisomerisierungskatalysator ein Molekularsieb umfaßt, das wenigstens einen Kanal mit einem kristallographisch freien Durchmesser längs den x- und/oder y-Ebenen der [001] Ansicht im Bereich von 0,42 bis 0,70 nm hat, wobei das Molekularsieb . vorzugsweise ein Zeolith mit der Ferrierit-Isotypstruktur ist.

14. verfahren zur Herstellung einer Zusammensetzung auf der Basis von verzweigten primären Alkoholen nach Anspruch 1, mit 13 bis 21 Kohlenstoffatomen, welches Verfahren die folgenden Stufen umfaßt:
a) Dimerisieren eines Olefineinsatzmaterials, das ein C₆-C₁₀-Olefin umfaßt, in Gegenwart eines homogenen Dimerisationskatalysators zur Ausbildung eines verzweigten C₁₂-C₂₀-Olefins; und
b) Umwandeln des verzweigten C₁₂-C₂₀-Olefins in die Zusammensetzung auf der Basis verzweigter primärer Alkohole.

15. Verfahren nach Anspruch 14, worin das Olefineinsatzmaterial wenigstens 90% lineare Olefine umfaßt.

16. Verfahren nach Anspruch 14 oder 15, worin das Olefineinsatzmaterial wenigstens 50% innere Olefine umfaßt.

17. Verfahren nach einem der Ansprüche 14 bis 16, worin der Dimerisationskatalysator eine Kombination aus einem Nikkelcarboxylat mit einem Alkylaluminiumhalogenid oder eine Kombination aus einem Nickelchelat mit einem Alkylaluminiumalkoxid umfaßt.

18. Verfahren nach einem der Ansprüche 12 bis 17, worin die Umwandlung des Olefins zu dem Alkohol in Stufe b) durch Hydroformylieren des Olefins mit Kohlenmonoxid und Wasserstoff in Gegenwart eines Hydroformylierungskatalysators vorgenommen wird.

19. Zusammensetzung auf der Basis verzweigter primärer Alkoholalkoxylate, erhältlich durch Umsetzen einer Zusammensetzung auf der Basis verzweigter primärer Alkohole nach einem der Ansprüche 1 bis 11 mit einer Oxiranverbindung.

20. Zusammensetzung auf der Basis verzweigter primärer Alkoholalkoxylate nach Anspruch 19, worin das Alkoxylat überwiegend Ethoxylat ist, erhältlich durch Umsetzen der Zusammensetzung auf der Basis primärer Alkohole mit Ethylenoxid.

21. Verzweigtes primäres Alkylsulfat, erhältlich durch Sulfatieren einer primären Alkoholzusammensetzung nach einem der Ansprüche 1 bis 11.

22. Verzweigtes alkoxyliertes primäres Alkylsulfat, erhältlich durch Alkoxylieren und Sulfatieren einer verzweigten primären Alkoholzusammensetzung nach einem der Ansprüche 1 bis 11.

23. Verzweigtes primäres Alkylcarboxylat, erhältlich durch Oxidieren einer verzweigten primären Alkoholzusammensetzung nach einem der Ansprüche 1 bis 11.

24. Tensidzusammensetzung, umfassend:
a) einen oder mehrere grenzflächenaktive Stoffe, ausgewählt aus der Gruppe von verzweigten primären Alkoholalkoxylaten nach Anspruch 19, verzweigten primären Alkylsulfaten nach Anspruch 21 und verzweigten alkoxylierten primären Alkylsulfaten nach Anspruch 22;
b) einen Gerüststoff; und
c) gegebenenfalls ein oder mehrere Additive, ausgewählt aus der Gruppe von Schaumunterdrückungsmitteln, Enzymen, Bleichmitteln, Bleichaktivatoren, optischen Aufhellern, Co-Gerüststoffen, Hydrotropen und Stabilisatoren.

25. Tensidzusammensetzung nach Anspruch 24, worin der Gerüststoff aus der Gruppe von Alkalimetallcarbonaten, Silikaten, Sulfaten, Polycarboxylaten, Aminocarboxylaten, Nitrilotriacetaten, Hydroxycarboxylaten, Citraten, Succinaten, substituierten und unsubstituierten Alkandi- und -polycarbonsäuren, komplexen Aluminosilikaten und deren Gemischen ausgewählt ist.

26. Tensidzusammensetzung nach Anspruch 24 oder 25, mit einem Gehalt an einem Bleichmittel, das aus der Gruppe von Perboraten, Percarbonaten, Persulfaten, organischen Peroxysäuren und deren Gemischen ausgewählt ist.

27. Tensidzusammensetzung nach einem der Ansprüche 24 bis 26, mit einem Gehalt an einem Bleichaktivator, der aus der Gruppe von Carbonsäureamiden, substituierten Carbonsäuren und Gemischen davon ausgewählt ist.

28. Tensidzusammensetzung nach einem der Ansprüche 24 bis 27, mit einem Gehalt an einem Hydrotrop, das aus der Gruppe von Alkalimetallsalzen von aromatischen Säuren oder Alkylcarbonsäuren, Alkalimetallchloriden, Harnstoff, Mono- oder Polyalkanolaminen und Gemischen davon ausgewählt ist.

29. Tensidzusammensetzung nach einem der Ansprüche 24 bis 28, ausgewählt aus der Gruppe von granulierten Waschmitteln, flüssigen Waschmitteln, Geschirrspülmitteln, Seifen, Shampoos und Scheuerreinigungsmitteln.

## Revendications

1. Composition d'alcool primaire ramifié, comportant de 11 à 36 atomes de carbone et un nombre moyen de ramifications par molécule de 0,7 à 3,0, ladite ramification comprenant des ramifications méthyle et éthyle, ladite composition comprenant moins de 0,5 atome % d'atomes de carbone quaternaire.

2. Alcool primaire ramifié suivant la revendication 1, dans lequel le nombre moyen de ramifications par molécule est de 1,0 à 3,0.

3. Composition d'alcool primaire ramifié suivant l'une ou l'autre des revendications 1 et 2, dans laquelle le nombre moyen de ramifications par molécule est de 1,5 à 2,3.

4. Composition d'alcool primaire ramifié suivant l'une quelconque des revendications 1 à 3, contenant moins de 5 % d'alcools linéaires.

5. Composition ramifiée suivant l'une quelconque des revendications 1 à 4, dans laquelle au moins 40 % du nombre de ramifications dans l'alcool sont des ramifications méthyle.

6. Composition d'alcool ramifié suivant l'une quelconque des revendications 1 à 5, dans laquelle de 5 % à 30 % du nombre de ramifications dans l'alcool sont des ramifications éthyle.

7. Composition d'alcool primaire ramifié suivant l'une quelconque des revendications 1 à 6, comportant de 5 à 25 % de ramification sur la position de carbone C₂, par rapport à l'atome de carbone de l'hydroxyle.

8. Composition d'alcool primaire ramifié suivant l'une quelconque des revendications 1 à 6, dans lequel de 10 à 20 % du nombre de ramifications sont en position C₂.

9. Composition d'alcool primaire ramifié suivant l'une quelconque des revendications 1 à 8, dans laquelle ladite composition a de 10 à 50 % du nombre de ramifications sur la position C₃

10. Composition d'alcool primaire ramifié suivant la revendication 9, dans laquelle ladite composition a de 15 à 30 % du nombre de ramifications sur la position C₃

11. Composition d'alcool primaire ramifié suivant l'une quelconque des revendications 1 à 10, dans laquelle ladite composition a au moins 5 % de type de ramification terminal isopropyle.

12. Procédé de préparation d'une composition d'alcool primaire ramifié suivant la revendication 1, lequel procédé comprend les étapes de :
a) mise en contact d'une alimentation oléfinique comprenant une oléfine linéaire comportant au moins 10 atomes de carbone avec un catalyseur efficace pour isomériser squelettiquement ladite oléfine linéaire et donner une oléfine ramifiée du même nombre de carbones; et
b) conversion de l'oléfine ramifiée précitée en la composition d'alcool primaire précitée.

13. Procédé suivant la revendication 12, dans lequel le catalyseur d'isomérisation squelettique comprend un tamis moléculaire comportant au moins un canal d'un diamètre libre cristallographique suivant les plans x et/ou y de la représentation [001] allant de 0,42 à 0,70 nanomètre, le tamis moléculaire étant avantageusement une zéolite ayant la structure isotypique de la ferriérite.

14. Procédé de préparation d'une composition d'alcool primaire ramifié suivant la revendication 1, comportant de 13 à 21 atomes de carbone, lequel procédé comprend les étapes de :
a) dimérisation, en présence d'un catalyseur de dimérisation homogène, d'une alimentation oléfinique comprenant une oléfine en C₆-C₁₀ pour donner une oléfine ramifiée en C₁₂-C₂₀; et
b) conversion de ladite oléfine ramifiée en C₁₂-C₂₀ en la composition d'alcool primaire ramifié précitée.

15. Procédé suivant la revendication 14, dans lequel l'alimentation oléfinique comprend au moins 90 % d'oléfines linéaires.

16. Procédé suivant l'une ou l'autre des revendications 14 et 15, dans lequel l'alimentation oléfinique comprend au moins 50 % d'oléfines internes.

17. Procédé suivant l'une quelconque des revendications 14 à 16, dans lequel le catalyseur de dimérisation précité comprend une combinaison d'un carboxylate de nickel avec un halogénure d'alkyl aluminium, ou une combinaison d'un chélate de nickel avec un alcoolate d'alkyl aluminium.

18. Procédé suivant l'une quelconque des revendications 12 à 17, dans lequel la conversion de l'oléfine en l'alcool dans l'étape b) est effectuée par hydroformylation de l'oléfine avec du monoxyde de carbone et de l'hydrogène, en présence d'un catalyseur d'hydroformylation.

19. Composition d'alcoxylat d'alcool primaire ramifié, préparable par la réaction d'une composition d'alcool primaire ramifié suivant l'une quelconque des revendications 1 à 11 avec un composé d'oxiranne.

20. Composition d'alcoxylat d'alcool primaire ramifié suivant la revendication 19, dans laquelle l'alcoxylat est en prédominance de l'éthoxylat, préparable par la réaction de la composition d'alcool primaire avec de l'oxyde d'éthylène.

21. Sulfate d'alkyle primaire ramifié, préparable par la sulfatation d'une composition d'alcool primaire suivant l'une quelconque des revendications 1 à 11.

22. Sulfate d'alkyle primaire alcoxylé ramifié, préparable par alcoxylation et sulfatation d'une composition d'alcool primaire ramifié suivant l'une quelconque des revendications 1 à 11.

23. Carboxylate d'alkyle primaire ramifié, préparable par oxydation d'une composition d'alcool primaire ramifié suivant l'une quelconque des revendications 1 à 11.

24. Composition détergente comprenant :
a) un ou plusieurs agents tensioactifs choisis dans le groupe comprenant les alcoxylates d'alcool primaire ramifié suivant la revendication 19, les sulfates d'alkyle primaires ramifiés suivant la revendication 21, et les sulfates d'alkyle primaires alcoxylés ramifiés suivant la revendication 22;
b) un adjuvant; et
c) éventuellement un ou plusieurs additifs choisis dans le groupe comprenant les agents de contrôle de mousse, les enzymes, les agents de blanchiment, les activateurs de blanchiment, les agents de brillantage optique, les coadjuvants, les hydrotropes et les stabilisants.

25. Composition détergente suivant la revendication 24, dans laquelle l'adjuvant est choisi dans le groupe comprenant les carbonates de métal alcalin, les silicates, les sulfates, les polycarboxylates, les aminocarboxylates, les nitrilotriacétates, les hydroxycarboxylates, les citrates, les succinates, les acides alcanedi- et polycarboxyliques substitués et non substitués, les aluminosilicates complexes et leurs mélanges.

26. Composition détergente suivant l'une ou l'autre des revendications 24 et 25, contenant un agent de blanchiment qui est choisi dans le groupe des perborates, percarbonates, persulfates, peroxyacides organiques et de leurs mélanges.

27. Composition détergente suivant l'une quelconque des revendications 24 à 26, contenant un activateur de blanchiment qui est choisi dans le groupe des amides d'acide carboxylique, acides carboxyliques substitués et de leurs mélanges.

28. Composition détergente suivant l'une quelconque des revendications 24 à 27, contenant un hydrotrope qui est choisi dans le groupe des sels de métal alcalin d'acides aromatiques ou d'acides alkyl carboxyliques, chlorures de métal alcalin, urée, mono- ou polyalcanolamines et de leurs mélanges.

29. Composition détergente suivant l'une quelconque des revendications 24 à 28, qui est choisie dans le groupe des détergents de blanchisserie granulaires, détergents de blanchisserie liquides, détergents de lave-vaisselle, savons, shampooings, et détergents de nettoyage.
